# EUROPEAN PATENT SPECIFICATION

(11) **EP 3 297 743 B1**
(45) Date of publication and mention of the grant of the patent: **16.09.2020**
(21) Application number: 16796874.2
(22) Date of filing: 30.03.2016
(51) Int. Cl.: B01D 24/22, B01D 29/085, B01D 39/02, B01J 20/281, G01N 1/40, G01N 33/487, G01N 30/06, G01N 30/60

(54) **SAMPLE EXTRACTION APPARATUS WITH MICRO ELUTION BED DESIGN**
PROBENAHMEVORRICHTUNG MIT MIKROELUIERUNGSBETTENTWURF
APPAREIL D'EXTRACTION D'ÉCHANTILLON AVEC LIT POUR MICROÉLUTION

(30) Priority: 20.05.2015 US 201514718065
(43) Date of publication of application: 28.03.2018
(73) Proprietor: Tecan SP, Inc., Baldwin Park CA 91706 (US)
(72) Inventor: DIMSON, Philip, Baldwin Park, CA 91706 (US)
(74) Representative: OK pat AG
(86) International application number: PCT/US2016/025100
(87) International publication number: WO 2016/186737

(56) References cited:
- WO-A1-98/00219
- WO-A1-2015/179579
- US-A1- 2002 151 086
- US-A1- 2007 102 358
- US-A1- 2007 102 358
- US-A1- 2007 117 222
- US-A1- 2009 223 893
- US-A1- 2010 140 173
- US-A1- 2010 140 173
- US-A1- 2011 180 482
- US-A1- 2012 175 368
- US-B1- 6 530 288
- US-B1- 8 247 545

## Description

The present invention relates to microcolumns for extraction of an analyte from a liquid sample, and particularly extraction of an analyte from biological fluids.

Accurate and inexpensive detection of analytes present in liquid samples, for example in biological fluids, such as blood and urine, is important to health care. Tests for analytes in blood and urine are conducted to monitor the health of patients, detect the presence of disease conditions, and monitor for the use of illegal or restricted drugs. For example, doctors, when administering drugs such as anti-arrythymics, asthmatic drugs, insulin, and anticoagulants, check the drug content of the blood to regulate the dosages of the patient. Drugs that can be abused, such as heroin, marijuana, cocaine, and codeine, can be tested to determine abuse of the drug, such as by employees and by athletes.

A technique used for detection of analytes includes selectively extracting the analyte from the biological fluid onto a solid media. The analyte is then removed from the solid media by a suitable elution liquid, and tests are conducted to determine whether the analyte is present in the eluent liquid. These tests are conducted using Gas Chromatography-Mass Spectrometry or Liquid Chromatography-Mass Spectrometry.

Extraction columns have been used in the past. For example, particulate silica has been used as the solid media in a column. In addition, media has been sandwiched between frits in a column with a single diameter cylindrical shape. Although these prior art devices can be effective, it is desirable to improve on these devices, and their impact in the overall process and their downstream environmental impact. It is desirable that the extraction device improve process throughput, remove a very high percentage of the analyte from the sample, be transportable, storable without damage, and be inexpensive. Moreover, it is desirable that any such device be compatible with existing automated equipment, and not leach into the biological or other fluid samples the eluent liquid or any compound that could interfere with the analytical results. Likewise, it is desirable to minimize the media bed volume and associated dead volumes to lower the volume of the wash eluent liquid. By minimizing the liquid volume, a more concentrated sample is obtained for analysis, and the sensitivity of the test is enhanced. High yields from the sample fluid with minimum elution volumes can be obtained by maintaining uniform flow through the extraction media, with no channeling and no dead volume.

US 8,247,545 discloses various microcolumns for the extraction of nucleic acids. US 2007/0102358 discloses a microcolumn for the extraction of analytes, comprising an upper flow distributor, an upper compression layer, a separation layer, a lower compression layer and a lower flow distributor.

### SUMMARY

The present invention is directed to an apparatus for extracting an analyte from a liquid sample according to claim 1, and a method for extracting an analyte according to claim 6. Further embodiments are provided in the dependent claims.

The present invention and its multiple embodiments are described further below. Of course, variations on these described embodiments will become apparent to those of ordinary skill in the art upon reading the present description, and thus, one of skill in the art would recognize suitable combinations and variations.

### BRIEF DESCRIPTION OF THE SEVERAL VIEWS OF THE DRAWINGS

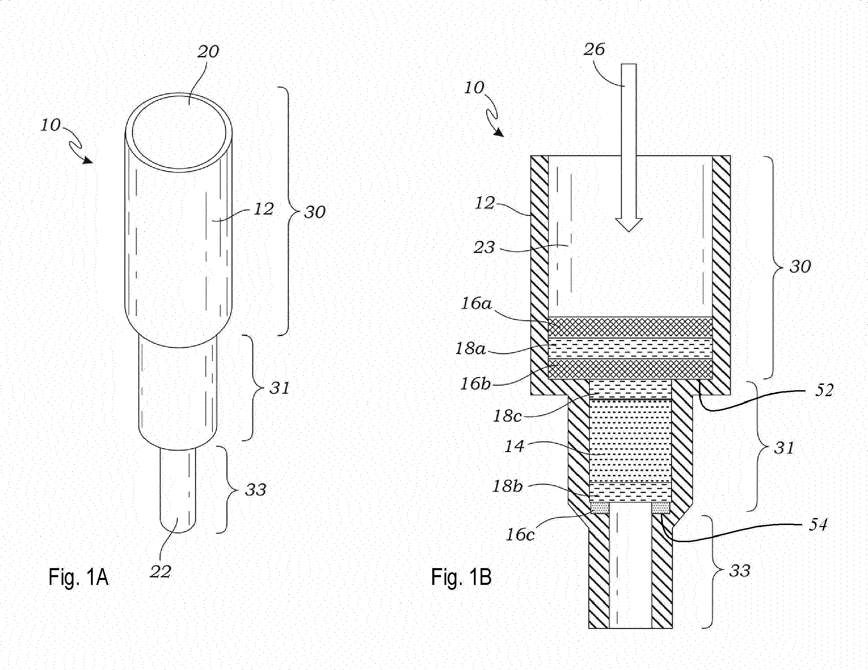

**FIG. 1A** is a schematic perspective view of the exterior of the present apparatus. **FIG. 1B** is an enlarged schematic cross-sectional side view of one embodiment of the layers used in the present apparatus.

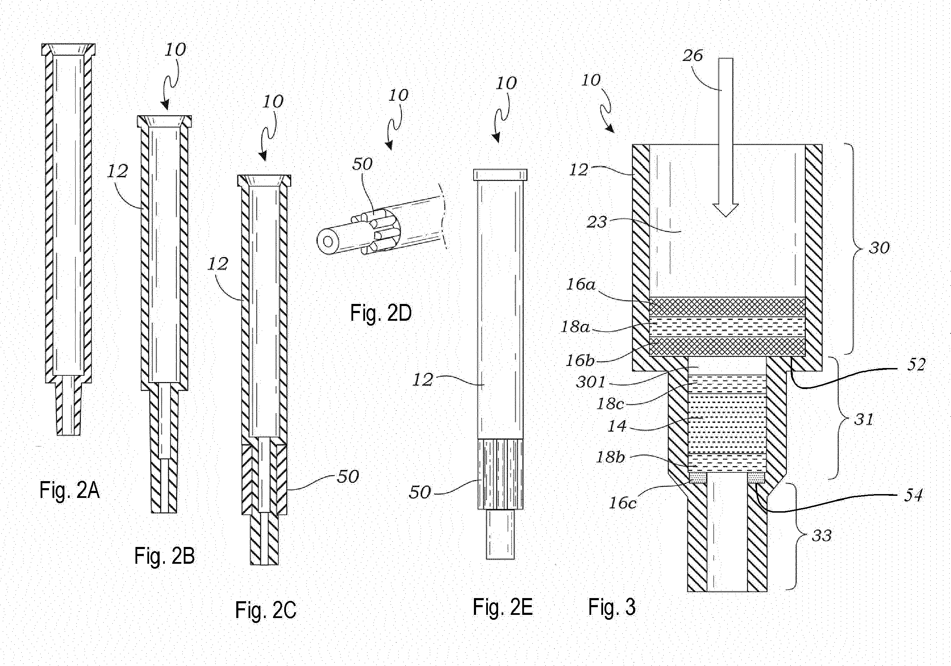

**FIG. 2A** is a cross-sectional side view of a conventional microcolumn **M.** **FIG. 2B** is a cross-sectional side view of an alternative embodiment of the present apparatus. **FIG. 2C** is a cross-sectional side view of a further alternative embodiment of the present apparatus with one or more ribs **50** on the exterior. **FIG. 2D** is a partial perspective view of the lower narrow diameter portion of the present apparatus, showing the ribs used in a luer tip system. **FIG. 2E** is an exterior side view of the present apparatus including the luer tip at the exit end thereof.
**FIG. 3** is a schematic cross-sectional side view of a further alternative embodiment of the layers used in the present apparatus which include an air gap.

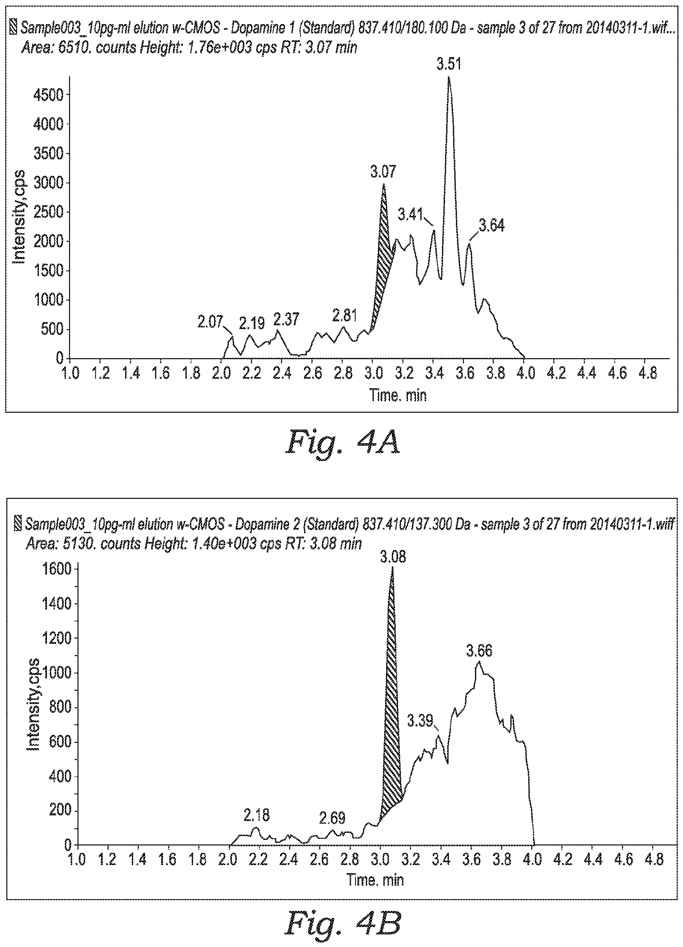

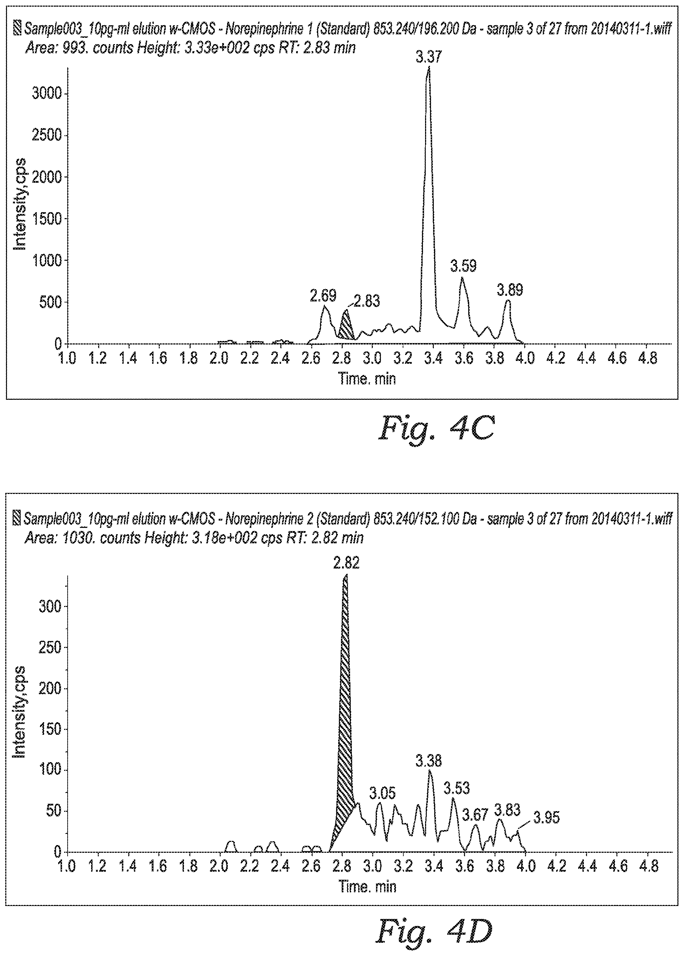

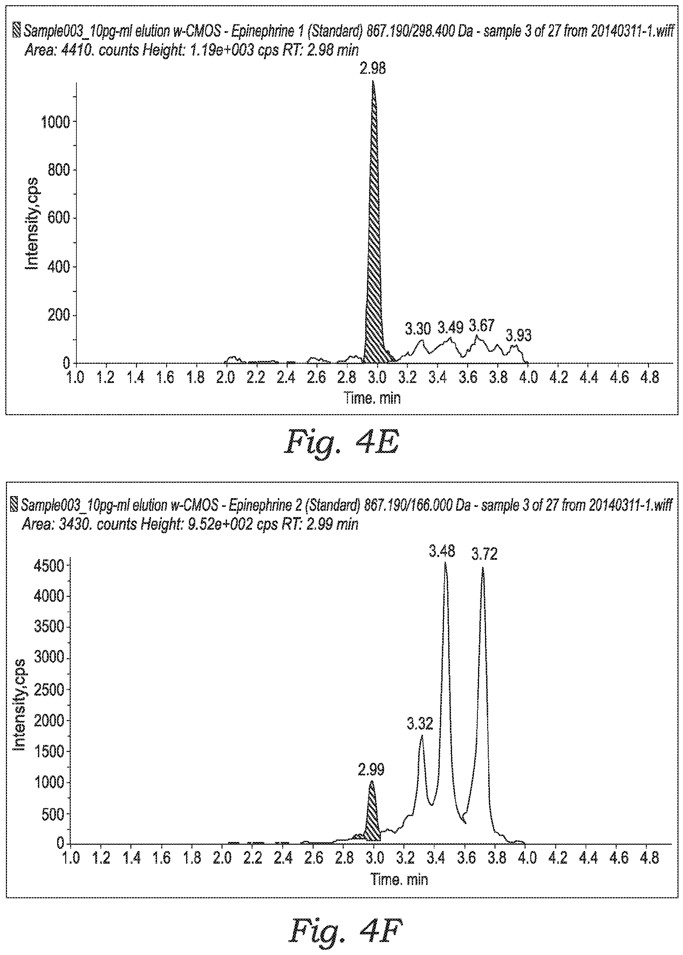

**FIG. 4A** is exemplary data obtained with the present apparatus using 10 pg/ml of dopamine. **FIG. 4B** is exemplary data obtained with the present apparatus using 10 pg/ml of dopamine. **FIG. 4C** is exemplary data obtained with the present apparatus using 10 pg/ml of norepinephrine. **FIG. 4D** is exemplary data obtained with the present apparatus using 10 pg/ml of norepinephrine. **FIG. 4E** is exemplary data obtained with the present apparatus using 10 pg/ml of epinephrine. **FIG. 4F** is exemplary data obtained with the present apparatus using 10 pg/ml of epinephrine.

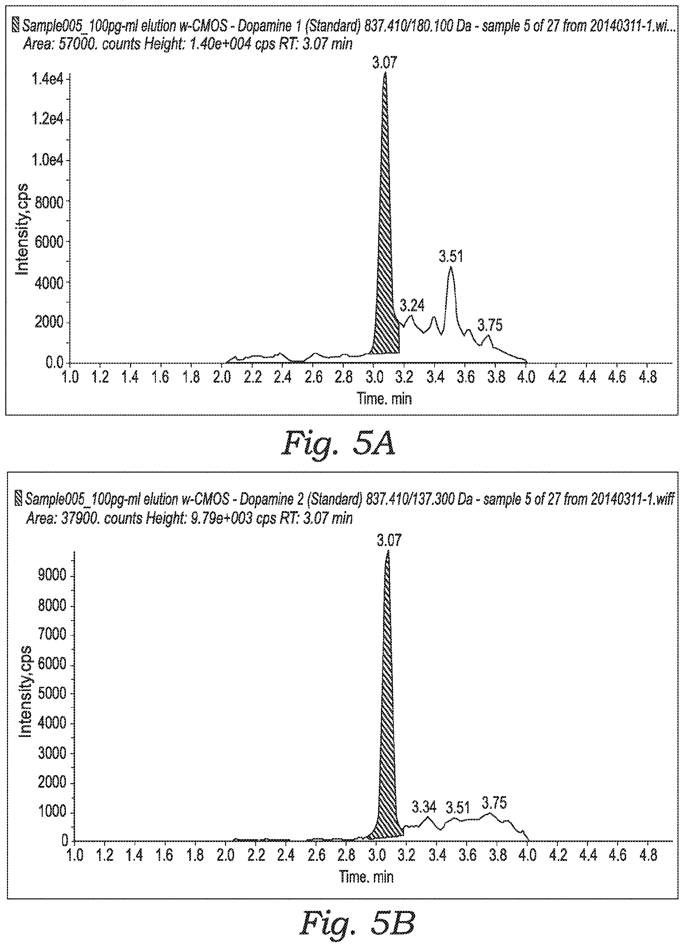

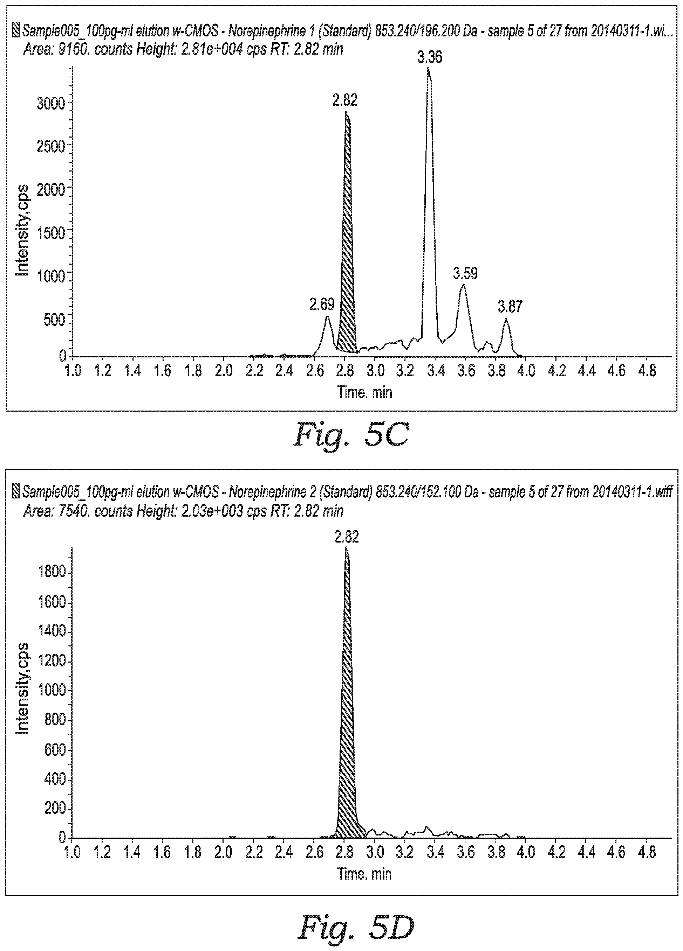

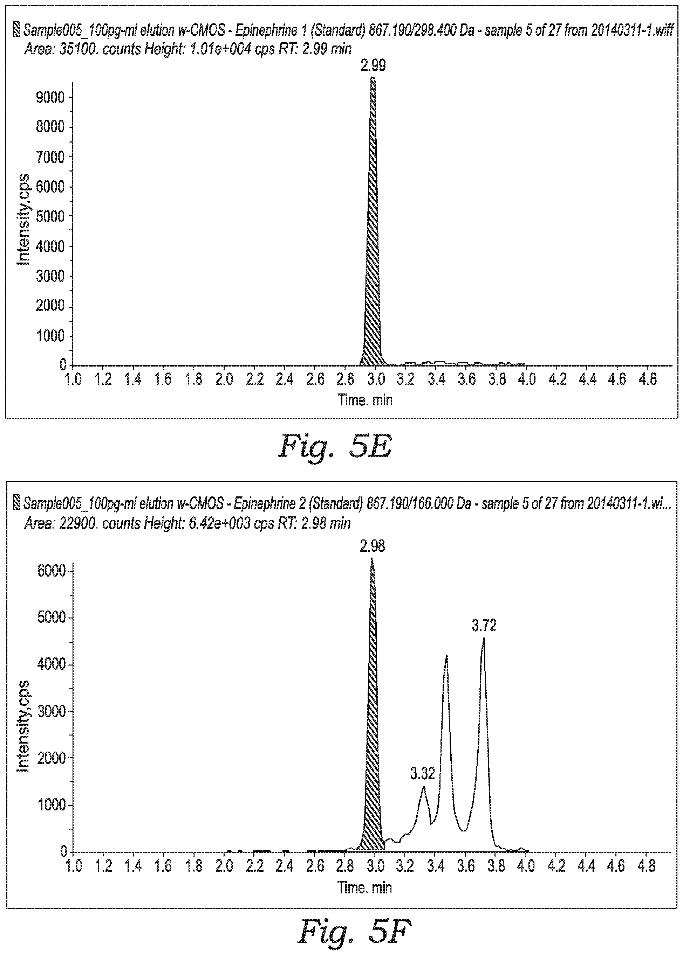

**FIG. 5A** is exemplary data obtained with the present apparatus using 100 pg/ml dopamine. **FIG. 5B** is exemplary data obtained with the present apparatus using 100 pg/ml of dopamine. **FIG. 5C** is exemplary data obtained with the present apparatus using 100 pg/ml of norepinephrine. **FIG. 5D** is exemplary data obtained with the present apparatus using 100 pg/ml of norepinephrine. **FIG. 5E** is exemplary data obtained with the present apparatus using 100 pg/ml of epinephrine. **FIG. 5F** is exemplary data obtained with the present apparatus using 100 pg/ml of epinephrine.

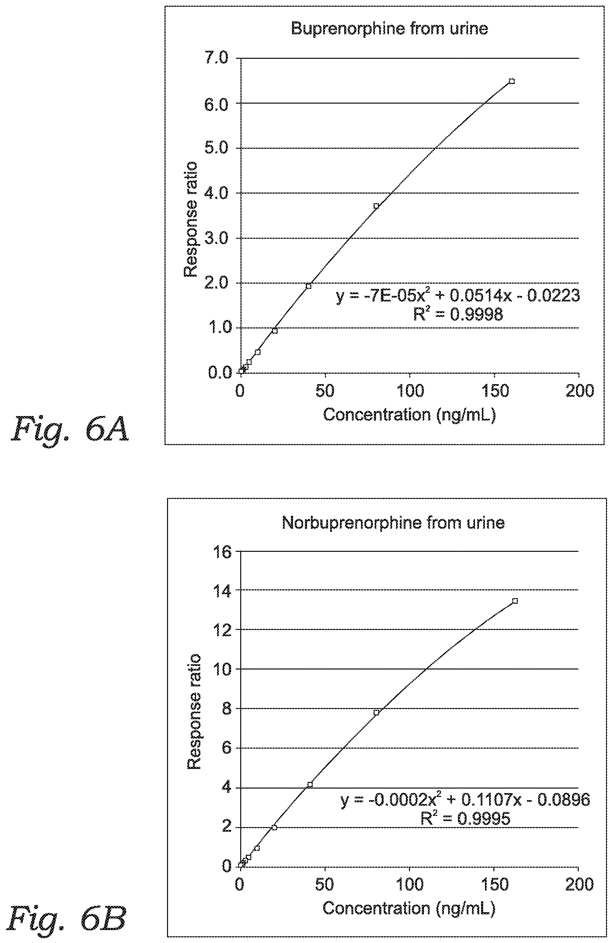

**FIG. 6A** is calibration curve of buprenorphine extracted from urine with the present apparatus. **FIG. 6B** is calibration curve of norbuprenorphine extracted from urine with the present apparatus.

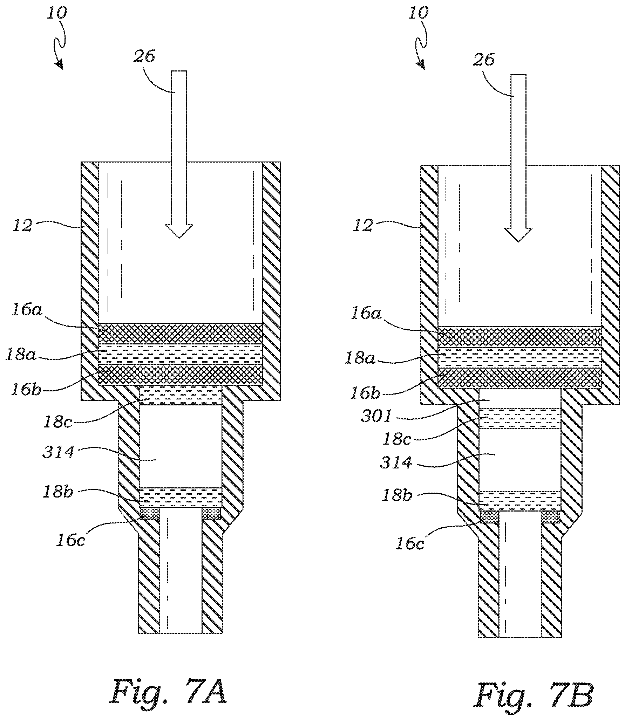

**FIG. 7A** is a schematic cross-sectional side view of a further alternative embodiment of the layers used in the present apparatus which include an air gap. **FIG. 7B** is a schematic cross-sectional side view of a further alternative embodiment of the layers used in the present apparatus which include multiple air gaps.

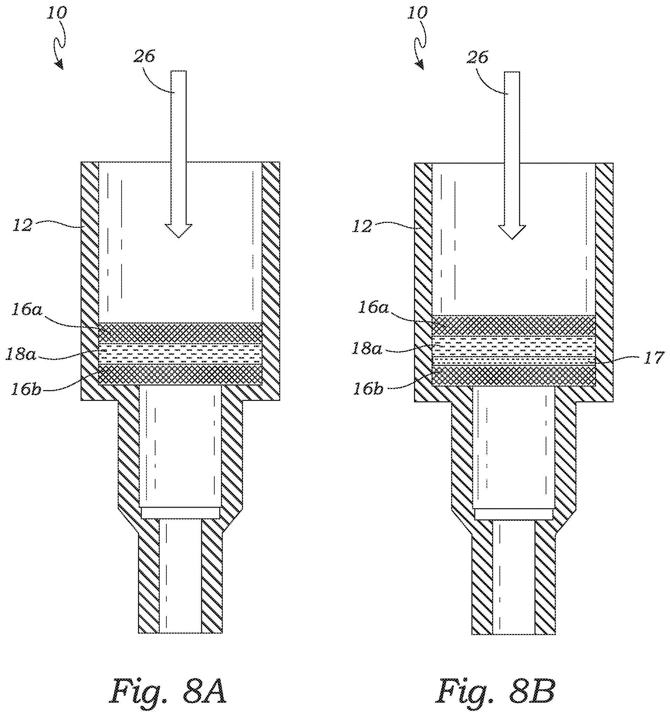

**FIG. 8A** is a schematic cross-sectional side view of a further alternative embodiment of the layers used in the present apparatus here included only in the upper full diameter bed region. **FIG. 8B** is a schematic cross-sectional side view of a further alternative embodiment of the layers used in the present apparatus again included only in the upper full diameter bed region, with an optional frit layer.

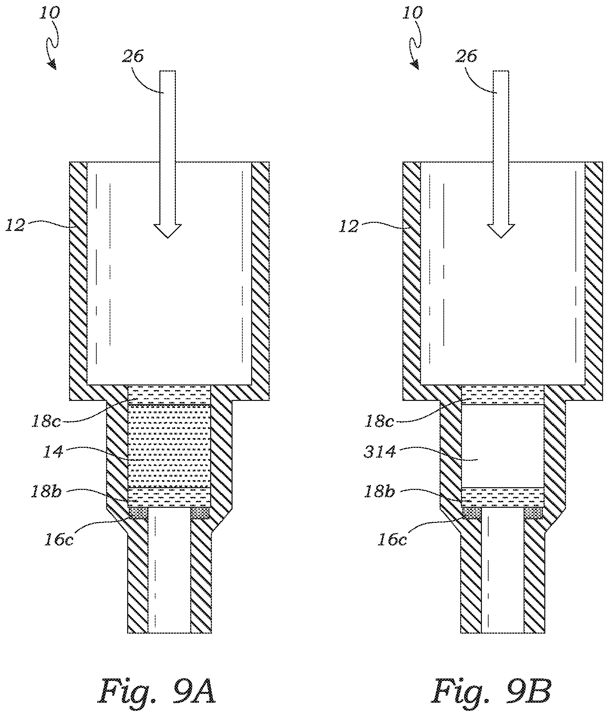

**FIG. 9A** is a schematic cross-sectional side view of a further alternative embodiment of the layers used in the present apparatus here included only in the intermediate reduced diameter bed region. **FIG. 9B** is a schematic cross-sectional side view of a further alternative embodiment of the layers used in the present apparatus again included only in the intermediate reduced diameter bed region, with an optional air gap.

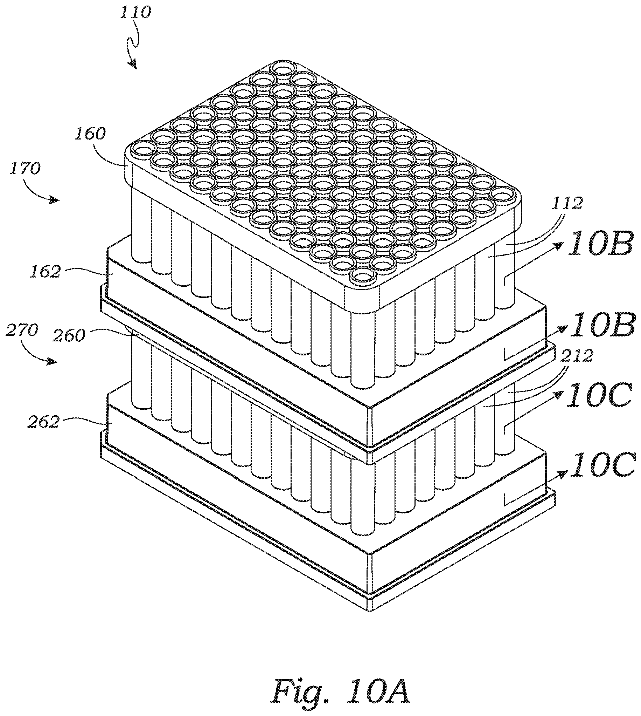

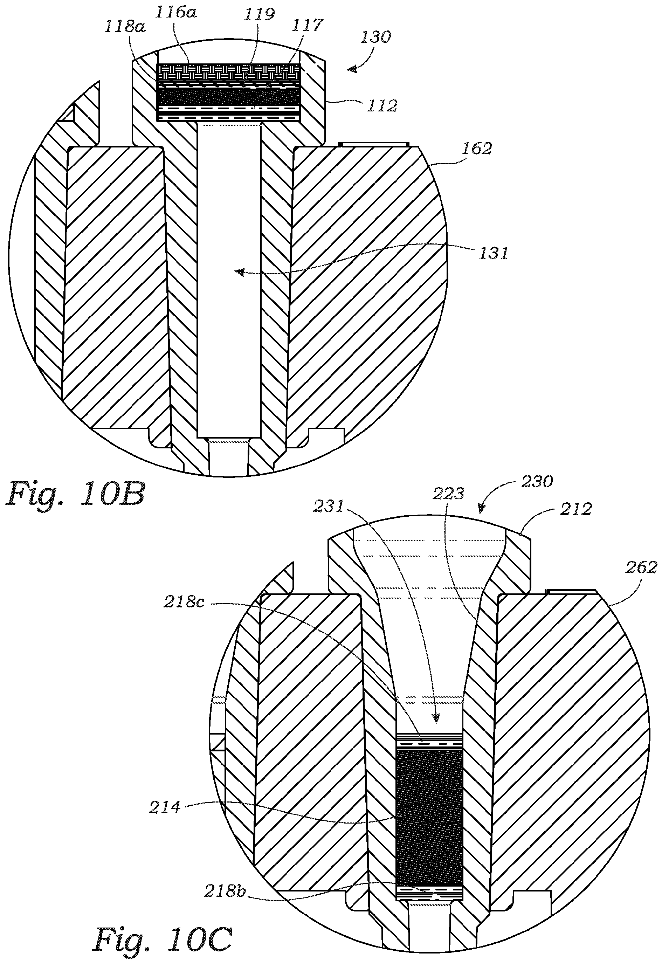

**FIG. 10A** is a perspective view of an exemplary embodiment of the present apparatus having dual barrels or configured in two stages. **FIG. 10B** is an enlarged partial cross-sectional side view of the upper barrel or first stage thereof. **FIG. 10C** is an enlarged partial cross-sectional side view of the lower barrel or second stage thereof.

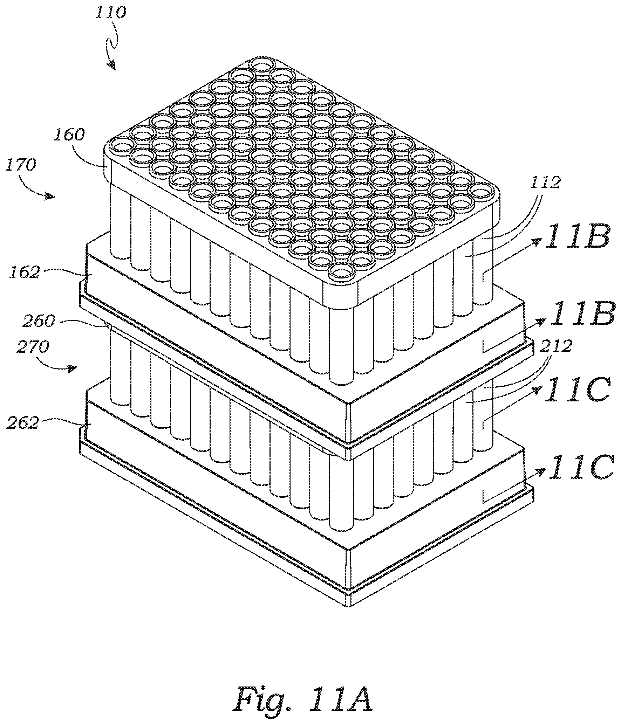

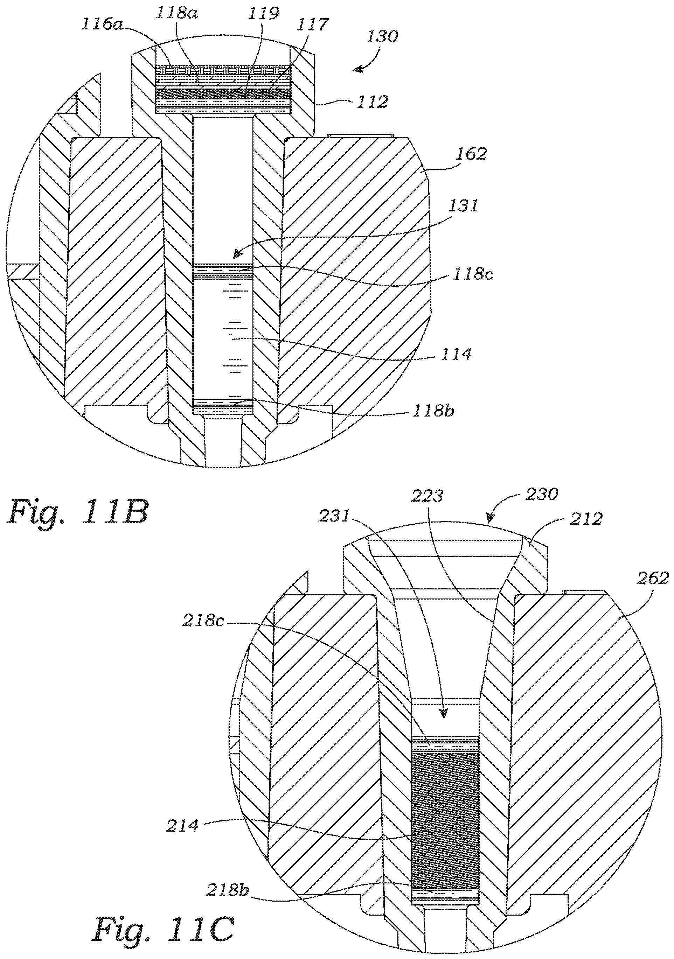

**FIG. 11A** is a perspective view of a further exemplary embodiment of the present apparatus having dual barrels or configured in two stages. **FIG. 11B** is an enlarged partial cross-sectional side view of the upper barrel or first stage thereof. **FIG. 11C** is an enlarged partial cross-sectional side view of the lower barrel or second stage thereof.

### DETAILED DESCRIPTION

The present invention is directed to an extraction apparatus that meets the needs of: improving downstream environmental impact and process throughput; removing a very high percentage of the analyte from the sample; transportability; storage without damage; price point; compatibility with existing automated equipment; leaching characteristics; minimization of the media bed volume and associated dead volumes; and enhancing sensitivity and uniformity of flow through the extraction media.

The present apparatus is useful for extracting an analyte from a liquid sample and comprises a container, typically a microcolumn, having an entrance, an opposed exit, and a passage therebetween for passage of a liquid sample containing an analyte therethrough.

Referring to **FIGS. 1A** and **1B****,** the microcolumn **12** of the present apparatus **10** has at least two regions in the passage **23** of the analyte: an area having a full diameter bed (aka the "full diameter bed region", shown in **FIGS. 1A** and **1B** as **30)** and an area having a reduced diameter bed (aka the "reduced diameter bed region", shown in **FIGS. 1A** and **1B** as **31**). As used herein, "diameter bed area" is measured by the surface area of a horizontal cross-section of the interior cavity of the container. Therefore, for a cylindrical container the diameter bed area is the area of the circle (*πr²*) whose diameter (= 2r) extends from one side of the interior cavity of the container to the other side of the interior cavity. The diameter bed area may also be referred to as the "effective area" of a particular layer.

Within the passage **23** in the reduced diameter bed region **31** is a layer of a microparticulate extraction media. Extraction media **14** may include any known sorbent and a variety of particles. The sorbent particles employed in the apparatus include any particulate matter that is capable of having at least one substance, either target or interfering, adhered thereto. Illustrative examples of sorbent particles that may be employed in the present invention include, but are not limited to: ion exchange sorbents; reversed phase sorbents; and normal phase sorbents. More particularly, the sorbent particles may be an inorganic material such as SiO₂ or an organic polymeric material such as poly(divinylbenzene). In some embodiments of the present invention, the sorbent particles may be treated with an organic functional group such as a C₂-C₂₂, preferably C₈-C₁₈ functional group. In one embodiment, the sorbent of the extraction media includes silica based particles (such as silica based carboxylic acids), diatomaceous earth particles, polymeric based particles, mono-dispersed silica and polymeric particles, and/or carbon graphite particles. The media is selected for extracting the analyte from the liquid sample. A suitable silica extraction media is described in U.S. Pat. No. 4,650,714. A preferred microparticulate silica extraction media is available from Avantor Performance Materials (previously known as J.T. Baker Chemical Company) of Phillipsburg, N.J., and is sold under their catalog number 7049-01.

The extraction media **14** has a small particle size having a number average particle size of less than about 40 microns, less than about 30 microns, less than about 25 microns, less than about 20 microns, less than about 15 microns, less than about 10 microns, or less than about 5 microns. In one embodiment, the extraction media has a number average particle size of less than about 20 microns, or more preferably less than about 10 microns. In addition, the extraction media does not have to be homogenous, but rather a different extraction media can be used in a single bed, or the apparatus can include multiple beds of extraction media for extracting different analytes from samples.

The extraction media **14** is sandwiched between at least two compression layers. In one embodiment, the diameter bed area of the extraction media is lower than the diameter bed area of the upper compression layer, such that the ratio of the effective area of the upper compression layer to the effective area of the extraction media layer is about 10 to 1, about 9 to 1, about 8 to 1 about 7 to 1 about 6 to 1, about 5 to 1, about 4 to 1, about 3 to 1, about 2 to 1, or about 1.5 to 1. In one embodiment, the ratio of the effective area of the upper compression layer to the effective area of the extraction media layer is about 4 to 1.

The extraction media may be loosely packed (where the sorbent is loosely sandwiched between the compression layers and free to move) or a compacted extraction media bed (where the extraction media is compacted between the two layers, or has relatively little additional possible space between particles).

The extraction media is sandwiched between either the upper compression layer **18a** and lower compression layer **18b** or the middle compression layer **18c** and lower compression layer **18b**, and compress the extraction media there between. Upper compression layer **18a** is located in full diameter bed region **30**, middle compression layer **18c** may be located in either the full diameter bed region **30** or reduced diameter bed region **31**, and lower compression layer **18b** is located in reduced diameter bed region **31.** In one embodiment only two compression layers are used. In one embodiment, at least three compression layers are used.

The compression layers are sufficiently porous that the liquid sample can flow therethrough, and are formed from a flexible material. One or more of the compression layers may be flat, spherical, or optimally shaped to suit the fluid flow through the apparatus (such as a truncated cone, prismatic, truncated pyramid, etc.). **FIG. 3** shows an embodiment where the middle and lower compression layers (**18c** and **18b**, respectively) are spherical. A mixture of shapes of compression layers may be used in a single microcolumn.

The narrow bore sandwich design pairs with the low dead volume column design for function. The chief purpose of compression layers **18** is to hold the extraction media **14** in place and compressed as a thin extraction layer. In one embodiment, one or more of the compression layer(s) has a pore size less than the particle size of the extraction media and functions as a flow rate limiter. They are sufficiently porous that the liquid sample can flow therethrough, and are composed of a flexible, hydrophilic material. The compression layer is preferably formed of a spongy, glass fiber, having no binder.

A suitable compression layer comprises a glass microfiber media made of analytically clean material. Suitable materials, which are available from Whatman Specialty Products, Inc. of Fairfield, N.J., include borosilicate glass fibers that are analytically clean and include no binder. This material, when purchased, has a smooth side and a rough side, where the smooth side is of lower porosity than the rough side. Preferably, it is the smooth side that is placed in contact with the microparticles of extraction layer **14.** Other suitable materials include frits and filters, such as polymer (e.g., polypropylene or polyethylene) frit materials. Said frits or filters may be cylindrical, die cut, or spherical, having a diameter to fit the interior cavity of the column; for instance, in the full diameter portion or in the reduced diameter portion.

Preferably compression layers **18** are resilient or "spongy" to hold the microparticles in place. In one aspect, the pore size for the compression layers is less than 10 microns, less than 5 microns, or less than 3 microns. Compression layers **18** generally are of the same thickness, having a thickness typically of from about 0.1 mm to about 3.25 mm, about 0.25 mm to about 3.25 mm, about 0.5 mm to about 3.0 mm, about 0.75 mm to about 3 mm, about 0.25 mm to about 2.5 mm, about 0.25 mm to about 2 mm, about 0.25 mm to about 1.5 mm, about 0.25 mm to about 1.25 mm, about 0.25 mm to about 1.0 mm, about 0.1 mm to about 0.75 mm, or about 0.1 mm to about 0.5 mm. In one aspect the compression layer(s) have a thickness of about 0.5 mm.

This is in contrast to the prior art microelution columns, which are made with cylindrical syringe barrel tubes, or funnel shaped sample reservoirs (see e.g., US 2006/0163163). Although some of these microelution columns have reduced dead volume after the bed, these designs do not address the ratio between the effective area of the upper compression layer compared to the effective area of the extraction media layer.

Reducing the effective area (πr²) of the extraction media bed about 4 - 1 in relation to the effective area of the upper compression layer leads to a corresponding reduced volume of sorbent material and reduced dead volumes for the reagent.

Flow distributors **16,** which are formed of a flexible mesh material, help provide uniform flow of the sample through the column, and physically retain the compression layers and microparticulate material in place in the column. Preferably, the mesh is 200 mesh or smaller, (i.e., has a mesh number of 200 or higher). In aspects of this embodiment, the mesh number is 150 or higher, 170 or higher, 200 or higher, 250 or higher, 270 or higher, 325 or higher, or 400 or higher. The mesh may be made of any flexible bio-inert material. It one embodiment it is made of Polyphenelyne Sulfide (PPS), Polytetrafluoroethylene (PTFE), Polyether ether Ketone (PEEK), Polyoxymethylene (POM), Ethylene Propylene Diene (EPDM), a Fluorinated elastomer (FKM), a Perfluoro elastomer (FFKM), polysulfone (PSU), Ethylene Tetrafluoroethylene (ETFE), Polypropylene (PP), (Poly)Chlorotrifluoroethylene (PCTFE/CTFE), polystyrene, high density polyethylene, polycarbonate, nylon, polyethylene terephthalate (PET), silicon, rubber, or polyester. In aspects of this embodiment it is made of polypropylene, or alternatively, polytetrafluoroethylene. A suitable material is available from Tetko, Inc. of Briarcliff Manor, N.Y., under catalog number 5-420134.

In one embodiment the microcolumn **12** also includes an upper mesh flow distributor **16a** above the upper compression layer **18a** for support, and, optionally, a middle flow distributor **16b** and/or a lower flow distributor **16c.** In one embodiment, the flow distributors may be layered or molded in the housing above and below the compression layer(s), sandwiching the compression layers **18** and the layer of extraction media **14** therebetween. The flow distributors **16** hold the extraction media **14** and the compression layers **18** in the reduced diameter bed region **31** of the microcolumn **12** and help distribute flow of the liquid sample to avoid channeling. As shown in the embodiment of **FIG. 1B****,** the upper flow distributor **16a** is located in the full diameter bed region **30,** the middle flow distributor **16b** is also located in the full-diameter bed region **30** and the lower flow distributor **16c,** if used, seats in the lower portion of the column in the reduced diameter bed region **31.** In one embodiment, the upper flow distributor **16a** is sized so that it is held in the bore of microcolumn **12** by a compression fit. Similarly, the other flow distributor layer(s) may also be sized for a compression fit.

Due to the combination of the narrow bore extraction media and the compression layer sandwich, rapid extraction of an analyte from a fluid can be obtained, with the apparatus being configured for very small volumes of elution liquid on the order of about 0.025 mL to about 0.25 mL, about 0.025 mL to about 0.2 mL, about 0.025 mL to about 0.15 mL, about 0.025 mL to about 0.100 mL, down from 0.5 mL to 1.5 mL. This smaller elution volume fits directly into autosampler trays for automation. Smaller volumes eliminate concentration steps and vial transfers and associated cross contamination fails. In addition, the extraction device of the present invention is inexpensive to use and manufacture, is stable during storage and transportation, and is compatible with existing automated equipment. The experimental data below displays the effective improvement in performance of a clinical assay done using regular Cerex columns versus the Narrow Bore version (the presently disclosed columns).

An apparatus **10** for extracting an analyte from a liquid sample is again shown in **FIG. 1A****.** As shown in **FIG. 1A****,** the apparatus **10** comprises the microcolumn **12,** which serves as a container for an extraction sandwich system. In one embodiment, microcolumn **12** has generally a tubular configuration, and has entrance **20,** opposed exit **22,** and passage **23** therebetween. Passage **23**, which is also referred to as a central bore, contains the extraction system. Passage **23** has two regions, upper full diameter bed region **30** and lower reduced diameter bed region **31.** The exit **22** may optionally be located in a separate region **33**, with a "tip" configuration.

The various layers may be located adjacent to each other, and may or may not be in direct contact with the surrounding layers. That is, with reference to **FIG. 3**, there may be one or more Air Gaps **301** between two layers which are deliberately located to prevent dripping or capillary flow and allow for the transfer of the column to an appropriate receiving vessel or plate. For instance, in one embodiment, there is an Air Gap layer **301** between the middle flow distributor **16b** and the middle compression layer **18c.** That is, in one embodiment, there may be an air gap between the layers that straddle the transition from the full diameter bed region **30** to the reduced diameter bed region **31.** In another embodiment there may be a strategic Air Gap **301** located after the lower compression layer **18b** but before the optional lower distributor layer **16c.**

In one embodiment, the Air Gap is positioned at or below the top edge of the reduced diameter bed region, and may have a height ranging from ½ of the diameter of the reduced diameter bed region to 4 times the diameter of the reduced diameter bed region. In aspects of this embodiment, the height of the air gap may be from, e.g., about ½ to about 1 times, about ½ to about 1.5 times, about ½ to about 2 times, about ½ to about 2.5 times, about ½ to about 3 times, about ½ to about 3.5 times, about ½ to about 4 times, about 1 to about 1.5 times, about 1 to about 2 times, about 1 to about 2.5 times, about 1 to about 3 times, about 1 to about 3.5 times, about 1 to about 4 times, about 1.5 to about 2 times, about 1.5 to about 2.5 times, about 1.5 to about 3 times, about 1.5 to about 3.5 times, about 1.5 to about 4 times, about 2 to about 2.5 times, about 2 to about 3 times, about 2 to about 3.5 times, about 2 to about 4 times, about 2.5 to about 3 times, about 2.5 to about 3.5 times, about 2.5 to about 4 times, about 3 to about 3.5 times, about 3 to about 4 times, or about 3.5 to about 4 times, the diameter of the reduced diameter bed region.

When placed strategically at the inlet to the reduced diameter bed region, the air gap prevents the unassisted capillary flow of certain classes of solvents or samples from bridging the gap for capillary transfer down to the next layer and then through the media bed. The air gap is particularly useful in areas with smaller diameters because the strength of the air gap is based on the surface tension between the liquid flowing through the apparatus and the gas located in the air gap.

The gap height may vary by the intended analytes to be tested, and might not be there at all. The Air Gap is particularly useful in a method where the compression layers are wet or preconditioned before the test sample is added to the microcolumn.

The structure of tip region **33** is not particularly limited and in one embodiment may be cylindrical or conical. Tip region **33** may have the same diameter as the reduced diameter bed region **31** or a smaller diameter than the reduced diameter bed region **31.** In one embodiment, the tip region **33** has the same footprint as the reduced diameter bed region **31**; in another embodiment, the footprint of the tip region **33** has a differently shaped footprint than the reduced diameter bed region **31.** In one embodiment, tip region **33** is optionally in the form of a luer tip or similar, which allows apparatus **10** to be used with conventional automated extraction apparatuses, which are designed to receive an extraction column having a luer tip.

In another embodiment, **FIG. 2C** provides a vertical cross-section of the present apparatus **10** with one or more ribs **50** on the exterior, though with all interior layers or features removed for simplicity. **FIG. 2D** is a partial perspective view of the lower narrow diameter portion of the present apparatus **10**, showing the ribs **50** used in a luer tip system. **FIG. 2E** is a side view of the present apparatus **10** including the luer tip system at the exit end of the present apparatus.

A liquid sample flows in the direction of arrow **26** shown in **FIG. 1B** through the passage **23.**

The portion of microcolumn **12** above the extraction sandwich system serves as a reservoir for the liquid sample, from which an analyte is to be extracted, and also a reservoir for a washing liquid, and an eluent liquid.

In one embodiment, the extraction system includes a four-layer sandwich construction with: (i) an upper flow distributor/support, (ii) an upper compression layer, (iii) an extraction layer, and (iv) a lower compression layer, where the upper flow distributer is located in the full diameter bed region and the upper compression layer, the extraction layer and the lower compression layer are located in the reduced diameter bed region.

In one embodiment, the extraction system is comprised of a five-layer sandwich construction, including (i) an upper flow distributor, (ii) a cylindrical or fabric compression layer, (iii) a spherical or cylindrical frit as a compression layer, (iv) the microparticulate extraction medium, and (v) a spherical or cylindrical frit as a lower compression layer. Layers (i)-(ii) would reside in the full diameter bed region, where layers (iii)-(v) would reside in the reduced diameter bed region.

In one embodiment, the extraction system is comprised of a six-layer sandwich construction, including (i) an upper flow distributor, (ii) a cylindrical or fabric compression layer, (iii) a lower flow distributor, (iv) a spherical or cylindrical frit as a compression layer, (v) the microparticulate extraction medium, and (vi) a spherical or cylindrical frit as a lower compression layer. Layers (i)-(iii) would reside in the full diameter bed region, where layers (iv)-(vi) would reside in the reduced diameter bed region. In one embodiment as shown in **FIG. 1B**, the extraction system is comprised of a seven-layer sandwich construction, that includes (i) upper flow distributor **16a,** (ii) upper compression layer **18a**, (iii) middle flow distributor **16b,** (iv) middle compression layer **18c** in the reduced diameter bed region **31**, (v) extraction layer **14** of microparticulate extraction medium, (vi) lower compression layer **18b**, and optionally (vii) lower flow distributor/support **16c** which may be molded as part of the container. In this embodiment, layers (i)-(iii) may be located in the full diameter bed region, and layers (iv)-(vii) may be located in the reduced diameter bed region. Alternatively, the division between full diameter and reduced diameter may occur between layers (ii) and (iii). Additional layers may be added.

In a further embodiment as shown in **FIG. 3**, the extraction system is comprised of an eight-layer sandwich construction, that includes: (i) upper flow distributor **16a,** (ii) upper compression layer **18a**, (iii) middle flow distributor **16b,** (iv) air gap layer **301**, (v) middle compression layer **18c** in the reduced diameter region **31**, (vi) extraction layer **14** of microparticulate extraction medium, (vii) lower compression layer **18b**, and optionally (viii) lower flow distributor/support **16c** which may be molded as part of the container. In this embodiment, layers (i)-(iii) may be located in the full diameter bed region, and layers (iv)-(viii) may be located in the reduced diameter bed region. Alternatively, the division between full diameter and reduced diameter may occur between layers (ii) and (iii). Additional layers may be added.

In a still further embodiment as shown in **FIG. 7A**, the extraction system is comprised of a seven-layer sandwich construction, that includes: (i) upper flow distributor **16a,** (ii) upper compression layer **18a**, (iii) middle flow distributor **16b,** (iv) middle compression layer **18c** in the reduced diameter region **31**, (v) air gap layer **314** in the place of extraction layer **14 (****FIGS. 1B** and **3**), (vi) lower compression layer **18b**, and optionally (vii) lower flow distributor/support **16c** which may be molded as part of the container. In this embodiment, layers (i)-(iii) may be located in the full diameter bed region, and layers (iv)-(vii) may be located in the reduced diameter bed region. Alternatively, the division between full diameter and reduced diameter may occur between layers (ii) and (iii). Additional layers may be added.

In a still further embodiment as shown in **FIG. 7B**, the extraction system is comprised of an eight-layer sandwich construction, that includes: (i) upper flow distributor **16a,** (ii) upper compression layer **18a**, (iii) middle flow distributor **16b,** (iv) air gap layer **301**, (v) middle compression layer **18c** in the reduced diameter region **31**, (vi) air gap layer **314** in the place of extraction layer **14** (**FIGS. 1B** and **3**), (vii) lower compression layer **18b**, and optionally (viii) lower flow distributor/support **16c** which may be molded as part of the container. In this embodiment, layers (i)-(iii) may be located in the full diameter bed region, and layers (iv)-(viii) may be located in the reduced diameter bed region. Alternatively, the division between full diameter and reduced diameter may occur between layers (ii) and (iii). Additional layers may be added.

In connection with the alternative embodiments of **FIGS. 7A** and **7B**, there may again be one or more Air Gaps **301**, **314** between two layers which are deliberately located to prevent dripping or capillary flow and allow for the transfer of the column to an appropriate receiving vessel or plate. For instance, in one such embodiment, there is an Air Gap layer **314** between the middle compression layer **18c** and the lower compression layer **18b** in the place of the extraction layer **14** (**FIGS**. **1B** and **3**), though it could be in addition to such extraction layer **14.** Or there is an Air Gap layer **301** between the middle flow distributor **16b** and the middle compression layer **18c** as well, so as to have effectively two air gaps separated by at least one material layer, further contributing to the prevention of dripping or capillary flow. That is, in one embodiment, there may be an air gap between the layers that straddle the transition from the full diameter bed region **30** to the reduced diameter bed region **31** and/or between the layers of the reduced diameter bed region **31.** In any such embodiments the height of thickness of any particular air gap, just as the material layers, can vary depending on the context, such that all drawings are to be understood as illustrative and not to scale and thus non-limiting.

In a still further embodiment as shown in **FIG. 8A**, the extraction system is comprised of a three-layer sandwich construction, that includes: (i) upper flow distributor **16a,** (ii) upper compression layer **18a**, and (iii) middle flow distributor **16b.** In this embodiment, layers (i)-(iii) may be located in the full diameter bed region, with no layers located in the reduced diameter bed region. Alternatively, the division between full diameter and reduced diameter may occur between layers (ii) and (iii). Additional layers may be added.

In a still further embodiment as shown in **FIG. 8B**, the extraction system is comprised of a four-layer sandwich construction, that includes: (i) upper flow distributor **16a,** (ii) upper compression layer **18a**, (iii) optional frit **17**, and (iv) middle flow distributor **16b.** In this embodiment, layers (i)-(iv) may be located in the full diameter bed region, with no layers located in the reduced diameter bed region. Alternatively, the division between full diameter and reduced diameter may occur between layers (iii) and (iv). Additional layers may be added.

According to the alternative exemplary embodiments of **FIGS. 8A** and **8B**, it will be appreciated that microcolumns **12** are shown having a first or upper extraction system or layered construction located in the upper full diameter bed region but no extraction system or layers in the lower reduced diameter bed region in which solid phase extraction or the like would have been conducted as in other embodiments herein. Such a reduced extraction system would have other clinical uses in single or dual stage applications, such as described below in connection with **FIG. 10****.**

In a still further embodiment as shown in **FIG. 9A**, the extraction system is comprised of a four-layer sandwich construction, that includes: (i) middle compression layer **18c** in the reduced diameter region **31,** (ii) extraction layer **14** of microparticulate extraction medium, (iii) lower compression layer **18b**, and optionally (iv) lower flow distributor/support **16c** which may be molded as part of the container. In this embodiment, layers (i)-(iv) may be located in the reduced diameter bed region, with no layers located in the full diameter bed region. Additional layers may be added.

And in a still further embodiment as shown in **FIG. 9B**, the extraction system is comprised of a four-layer sandwich construction, that includes: (i) middle compression layer **18c** in the reduced diameter region **31**, (ii) air gap layer **314** in the place of extraction layer **14** (**FIG. 9A**), (iii) lower compression layer **18b**, and optionally (iv) lower flow distributor/support **16c** which may be molded as part of the container. In this embodiment, layers (i)-(iv) may again be located in the reduced diameter bed region, with no layers located in the full diameter bed region. Additional layers may be added.

According to the alternative exemplary embodiments of **FIGS. 9A** and **9B**, it will be appreciated that microcolumns **12** are shown having a second or lower extraction system or layered construction located in the lower reduced diameter bed region but no extraction system or layers in the upper full diameter bed region in which the upper or middle flow distributor layers **16a**, **16b** or the like would have been positioned as in other embodiments herein. Such a reduced extraction system would have other clinical uses in single or dual stage applications, such as described below in connection with **FIGS. 10** and **11****.**

All of the components of apparatus **10** are made of materials that are substantially inert to biological fluids so that when a biological fluid such as blood or urine is passed through apparatus **10,** substantially nothing passes from apparatus **10** into the blood or urine. In one embodiment, microcolumn **12** is made of a biologically inert material. In one aspect, the biologically inert material is a plastic. In aspects of this embodiment, the biologically inert material is a fluorinated polymer or polypropylene. In other aspects the material is Polyphenelyne Sulfide (PPS), Polytetrafluoroethylene (PTFE), Polyether ether Ketone (PEEK), Polyoxymethylene (POM), Ethylene Propylene Diene (EPDM), a Fluorinated elastomer (FKM), a Perfluoro elastomer (FFKM), polysulfone (PSU), Ethylene Tetrafluoroethylene (ETFE), Polypropylene (PP), (Poly)Chlorotrifluoroethylene (PCTFE/CTFE), polystyrene, high density polyethylene, polycarbonate, nylon, or polyethylene terephthalate (PET), silicon, rubber, polyester, or ceramics.

A typical microcolumn according to the present invention has an internal diameter of about 0.01 inch to about 2 inches, about 0.025 inches to about 1.75 inches, about 0.05 inches to about 1.5 inches, of about 0.075 inches to about 1.25 inches, of about 0.1 inches to about 1 inch. In other aspects of this embodiment, the internal diameter is at least 0.01 inch, at least 0.025 inches, at least 0.05 inches, 0.075 inches, at least 0.1 inch, at least 0.25 inches, at least 0.5 inches, at least 0.75 inches, at least 1 inch, at least 1.25 inches, at least 1.5 inches, at least 1.75 inches, or at least 2 inches. In still other aspects of this embodiment, the internal diameter is at most 0.01 inch, at most 0.025 inches, at most 0.05 inches, 0.075 inches, at most 0.1 inch, at most 0.25 inches, at most 0.5 inches, at most 0.75 inches, at most 1 inch, at most 1.25 inches, at most 1.5 inches, at most 1.75 inches, or at most 2 inches. In a preferred embodiment the microcolumn has an internal diameter of about 0.1 inch to 1.0 inch.

A typical microcolumn according to the present invention has a length, excluding the tip if present, of about 0.25 inches to about 5 inches, 0.5 inches to about 4.5 inches, 0.5 inches to about 4 inches, 0.5 inches to about 3.5 inches, about 0.5 inches to about 3 inches, about 0.5 inches to about 2.5 inches, about 0.5 inches to about 2 inches, about 0.5 inches to about 1.5 inches, about 0.5 inches to about 1 inch, about 1.75 inches to about 3 inches, about 2 inches to about 3 inches, about 2.5 inches to about 3 inches. In other aspects of the invention, the microcolumn has a length of at least 0.25 inches, at least 0.5 inches, at least 0.75 inches, at least 1 inch, at least 1.25 inches, at least 1.5 inches, at least 2 inches, at least 2.25 inches, at least 2.5 inches, at least 2.75 inches, at least 3 inches, at least 3.25 inches, at least 3.5 inches, at least 3.75 inches, at least 4 inches, at least 4.25 inches, at least 4.5 inches, at least 4.75 inches, or at least 5 inches. In still further aspects, the microcolumn has a length, excluding the tip if present of at most 0.25 inches, at most 0.5 inches, at most 0.75 inches, at most 1 inch, at most 1.25 inches, at most 1.5 inches, at most 2 inches, at most 2.25 inches, at most 2.5 inches, at most 2.75 inches, at most 3 inches, at most 3.25 inches, at most 3.5 inches, at most 3.75 inches, at most 4 inches, at most 4.25 inches, at most 4.5 inches, at most 4.75 inches, or at most 5 inches. In a preferred embodiment, the microcolumn has a length, excluding the tip of about 0.5 inches to about 3 inches. The length of the tip (if present) is not particularly limited but may range from about 0.1 inch to 1 inch.

The upper full diameter bed region generally includes at least about 40%, 45%, 50%, 55%, 60%, 65%, 70%, 75%, 80%, or 85% of the total microcolumn length. In one embodiment, the upper full diameter bed region includes at least about 75% of the total microcolumn length.

The microcolumn of the apparatus need not have the shape shown in the figures. For example, it need not be cylindrical, and may instead have a square footprint, a polygonal footprint (e.g., hexagonal, octagonal, etc.), tubular, or include combinations of multiple shapes. As used herein "footprint" describes the shape of a horizontal cross-section of the interior cavity (i.e., passage 23) of the microcolumn. In one embodiment, the full diameter bed region has a first footprint shape, and the reduced diameter bed region has a second footprint shape, and the tip (if present) may have a third footprint shape (or may have the same footprint shape as either the full diameter bed region or the reduced diameter bed region).

In addition, in one embodiment of the invention, entrance **20** can be designed or configured to receive a connective fitting to connect the microcolumn to a fluid input device. Such connective fittings include luer tips, luer-lock extensions or tapers of various types, male and female-type connections of various types, threaded connections, or barb connections.

As used herein, a "fluid input device" is any device which is in contact with the entrance and directly transfers the sample or reagent to entrance **20** in a connected manner. Fluid input devices may include automated fluid dispensing systems, automated liquid handling platforms, syringes, and microdispensers. The fluid input device may dispense the sample or reagent to the microcolumn automatically, semi-automatically, or manually. For instance a fluid input device may include a reservoir containing a liquid sample, which once connected to the entrance, can automatically dispense sample to the microcolumn.

The narrow bore sandwich layer in the reduced diameter region can be assembled as a stand-alone column barrel design or patterned in a one-piece block format for automated processing.

The present apparatus may be in a single column format, which is convenient and cost effective for preparing a small number of samples, or a multi-column array or format (aka, an "extraction plate"), which is suited for preparing large numbers of samples in parallel.

One embodiment of the invention is an extraction plate configured for or containing the narrow bore columns discussed above. This extraction plate may be a molded plate containing a plurality of columns. The columns may be arrayed to align or intercalate with the wells of conventional "multi-well" formats, such that each column would elute into a well in a standard (or custom) multi-well plate. Multi-well formats are commonly used with robotic fluid dispensing systems, such as autosamplers. Typical multi-well formats are not limited, but include 48-, 96-, and 384- and 1,584-well standard plate formats.

Fluids are usually forced through the present apparatus and into the collection containers (or the wells of a "collection tray" or "plate"), either by drawing a vacuum across the device with a specially designed vacuum manifold, or by using centrifugal or gravitational force. In specially designed vacuum manifold systems, both the column and the receiving means (such as a well or collection tube) may be integrated into the vacuum system to optimize extraction. Centrifugal force is generated by placing the apparatus, together with a suitable collection tube or tray, into a centrifuge specifically designed for the intended purpose. However, in one embodiment gravity may be sufficient to force the fluid through the present apparatus as well.

The present apparatus may use conventional collection containers or collection plates, such as glass tubes, centrifuge tubes, Eppendorf tubes, or standard multi-well plates or trays. The present apparatus may alternately use collection containers specifically designed to be compatible with the present extraction plate.

Methods of using the present invention include extraction of an analyte from a test sample. A test sample refers to any sample that may contain an analyte of interest. A test sample may be a biological sample, that is, a sample obtained from any biological source, such as an animal, a plant, a fungus, a microorganism, a cell culture, an organ culture, etc. In aspects of this embodiment, a biological sample includes a blood sample including a whole blood sample, a plasma sample, or a serum sample, a saliva sample, a urine sample, cerebrospinal fluid sample, a bile sample, a tissue sample, or any other sample that can be obtained, extracted or isolated from a biological source. Such biological samples may be obtained, for example, from a patient; that is, a living person, male or female, presenting oneself in a clinical setting for diagnosis, prognosis, or treatment of a disease or condition. In one embodiment, the sample is obtained from a patient, for example, a plasma specimen. The plasma specimen may be taken with or without the use of anticoagulants.

A test sample may be an environmental sample. Environmental samples are samples taken from dirt, plant matter, or fluid sources (such as ground water, oceans, or rivers etc.). Dirt (aka "soil samples") may be taken from agricultural sites or sites of environmental interest and may have the analyte extracted, including the removal of particulate matter.

The methods of using the present apparatuses include contacting the apparatus with the sample in a liquid buffer, and eluting the sample from the apparatus with an elution buffer. Further steps might include conditioning the apparatus and washing steps (before, during, or after contacting the apparatus with a sample).

The present apparatus may also be included in a kit specifically designed to capture and separate a particular analyte of interest. The microparticulate extraction media may be specifically adapted to separate specific analytes of interest. The kit may include suitable reagents (labels, washes, etc.), buffers or elution buffers, in concentrated form or in a form suitable for direct use. The kit may also include an extraction plate or array of columns as described above, and accompanying couplings to connect the plate to fluid dispensing devices and/or plates or vials for receiving the eluted solution containing the sample.

Turning next to **FIGS. 10A-10C** there is shown a further exemplary embodiment of an apparatus **110** for extracting an analyte from a liquid sample, here the apparatus **110** configured as a multi-column array or format (aka, an "extraction plate") of microcolumns **112**, **212** in series as by stacking or nesting one array of microcolumns on or over another, which arrangement is suited for preparing large numbers of samples in parallel and further for multi-stage extraction or elution as described further below. Here, there are thus formed an upper first array **170** of first microcolumns **112** and a lower second array **270** of second microcolumns **212.** In the illustrated embodiment, each array **170**, **270** comprises a plurality of microcolumns **112**, **212** maintained in a substantially parallel and offset pattern or arrangement as by a respective top plate **160, 260** and base **162, 262** - as illustrated, there are ninety-six (96) such microcolumns **112**, **212** per array **170**, **270**, though it will be appreciated that such is merely exemplary. The nesting of respective first and second microcolumns **112**, **212** may be achieved employing any appropriate technique now known or later developed. However, while a tight or interference fit or substantially sealed arrangement as typically configured may be employed in nesting the first and second microcolumns **112**, **212** in series where the sample is drawn through both microcolumns **112**, **212** simultaneously as by applying pressure or a vacuum or by centrifugal or gravitational force, uniquely according to aspects of the present invention a loose or unsealed nesting of the first and second microcolumns **112**, **212** is to be employed so as to allow pressurizing the upper or first microcolumns **112** from the top without yet passing the liquid through to the lower or second microcolumns **212**, more about which is said below. Notably, the configuration of the exit **22** or tip region **33** and/or the reduced diameter bed region **31** (**FIG. 1A**) of the first microcolumn **112** is such that there is a loose fit or gaps left when nesting in the entrance **20** at the full diameter bed region **30** of the second microcolumn **212** so as to allow venting and thus pressurization from above and activation of the first microcolumn **112** without necessarily activating, pressurizing, or flowing through the second microcolumn **212.**

Referring to **FIG. 10B**, there is shown an enlarged partial cross-sectional view of an upper or first microcolumn **112** associated with the upper or first stage or array **170** of the apparatus **110**, as taken along section line **10B-10B** of **FIG. 10A****.** Similar to the exemplary embodiments of **FIGS. 8A** and **8B**, the first microcolumn **112** is shown as having a first or upper extraction system or layered construction located in the upper full diameter bed region **130** but no extraction system or layers in the lower reduced diameter bed region **131.** Specifically, the upper first layered construction is comprised of: (i) upper flow distributor **116a** configured as a screen or mesh, (ii) upper compression layer **118a** such as a glass fiber filter material, (iii) optional sorbent or extraction layer **119**, and (iv) optional frit **117.** Again, additional layers may be added or substituted, it being noted, for example, the alternative position of the optional frit **117** at the bottom of the layers rather than at an intermediate position and of an upper sorbent **119** rather than a second flow distributor layer (compare to middle flow distributor **16b** of **FIGS. 1B****,** **3****,** **7A, 7B****,** **8A** and **8B**).

And in **FIG.** 10C there is shown an enlarged partial cross-sectional view of a lower or second microcolumn **212** associated with the lower or second stage or array **270** of the apparatus **110** shown in **FIG. 10A**, as taken along section line **10C-10C of** **FIG. 10A****.** Similar to the exemplary embodiments of **FIGS. 9A** and **9B**, the second microcolumn **212** is shown as having a second or lower extraction system or layered construction located in the lower reduced diameter bed region **231** but no extraction system or layers in the upper full diameter bed region **230.** Specifically, the lower second layered construction is comprised of: (i) middle compression layer **218c**, (ii) extraction layer **214** of microparticulate extraction medium, and (iii) lower compression layer **218b.** Again, additional layers may be added or substituted, here it being noted, for example, that the lower and middle compression layers **218b**, **218c** may be frits or any other such materials bounding the extraction layer **214** and that the optional lower flow distributor/support **16c (****FIGS. 9A** and **9B**) which may be molded as part of the container is not included in the alternative exemplary lower or second microcolumn **212** associated with the second stage or array **270** of the apparatus **10.** As also shown in **FIG. 10C**, optionally, microcolumn passage **223** may be tapered from the upper full diameter bed region **230** to the reduced diameter bed region **231**, which it will be appreciated assists in transitioning the sample or elution liquid from one bed region to the other.

More generally, with continued reference to **FIGS. 10A-10C****,** it will be appreciated that by configuring the apparatus **110** in two stages with two separate microcolumns **112**, **212** in series, the upper first microcolumn **112** being configured with a layered construction in its upper full diameter bed region **130** and the lower second microcolumn **212** being configured with a layered construction in its lower reduced diameter bed region **231**, there is achieved a multi-stage extraction system somewhat analogous to doing so in a single microcolumn **12** having two distinct bed regions and related layered constructions, or effectively two stages, but by doing so in two microcolumns **112**, **212** in series more flexibility in use is beneficially derived. For example, by physically separating the upper and lower extraction or filtration systems between the two microcolumns **112**, **212**, larger volumes or spaces therebetween are formed, though with less dead spaces, both to serve as functional reservoirs for samples or elution liquids and effectively forming air gaps to prevent or mitigate against dripping or capillary flow and to allow for the transfer of the one or more columns between each other or to an appropriate receiving vessel or plate. In that regard, there is thus provided new functional utility and increased fields of use. In one embodiment, such a single-or multi-stage microcolumn alone or in an array functions as a test tube or incubation device, wherein a self-contained system is provided in which aqueous solutions will not readily leak out based on supported surface tension and other such factors yet with the ability to also serve as an effective sold phase extraction device. Furthermore, in the particular exemplary embodiment wherein the upper or first microcolumn **112** associated with the first stage comprises the optional sorbent or extraction layer **119**, it will be appreciated that there is thus provided a true dual- or two-stage solid phase extraction apparatus **110.** Those skilled in the art will appreciate that a variety of other arrangements of the microcolumns **112**, **212** and the layered constructions within each are possible without departing from the scope of the invention. By way of further illustration and not limitation, when the first and second microcolumns **112**, **212** are configured as set forth herein so as to nest in an unsealed or vented fashion, it will be appreciated that when a sample is first filtered or extracted in the upper or first stage microcolumns **112** pressure is applied thereto from above so as to in this example push the sample through the first or upper extraction system or layered construction located in the upper full diameter bed region **130** of each and have the sample flow downwardly and collect within the passage **223** of the respective lower or second microcolumns **212** above the second or lower extraction system or layered construction located in the lower reduced diameter bed region **231** of each lower second microcolumn **212** without flowing or being forced therethrough, again because the pressure applied to the upper first microcolumns **112** is vented. Once the sample is prepared as through filtration or extraction via the first microcolumns **112**, the upper or first array **170** of microcolumns 112 may simply be removed or jettisoned and then further processing conducted on the lower or second array **270** of microcolumns **212**, as by then pressurizing the second microcolumns **212** and/or employing an elution solvent to release the analytes of interest. It will be appreciated that a variety of mechanisms for manually, semi-automatically or automatically selectively activating or filtering with the upper first array **170** of microcolumns **112** and then the lower second array **270** of microcolumns **212**, whether now known or later developed, are possible according to aspects of the present invention, which will be further appreciated by the further discussion and examples herein. It will also be appreciated that while the second stage filtration or extraction activation may be accomplished using pressure, centrifuge or gravity or other such techniques now known or later developed to draw the sample through the second microcolumns **212** may also be employed without departing from the scope of the present invention.

Referring next to **FIGS. 11A-11C** there is shown a still further exemplary embodiment of an apparatus **110** for extracting an analyte from a liquid sample similar to that of **FIGS. 10A-****10C** with the apparatus **110** again configured as a multi-column array or format (aka, an "extraction plate") of microcolumns **112**, **212** in series as by stacking or nesting one array of microcolumns on or over another. Once more, there are thus formed an upper first array **170** of first microcolumns **112** and a lower second array **270** of second microcolumns **212.** In the illustrated embodiment, each array **170**, **270** comprises a plurality of microcolumns **112**, **212** maintained in a substantially parallel and offset pattern or arrangement as by a respective top plate **160, 260** and base **162, 262** - as again illustrated, there are ninety-six (96) such microcolumns **112**, **212** per array **170**, **270,** though it will be appreciated that such is merely exemplary. The nesting of respective first and second microcolumns **112**, **212** may again be achieved employing any appropriate technique now known or later developed, including sealed (tight) and unsealed (loose) fit arrangements, though there again being advantages in the present multi-stage arrangement and micro elution bed configuration to loosely nesting the respective microcolumns **112**, **212** so as to vent therebetween.

As shown in the enlarged partial cross-sectional view of an upper or first microcolumn **112** associated with the upper or first stage or array **170** of the apparatus **110** of **FIG. 11B**, here as taken along section line **11B-11B** of **FIG. 11A**, similar to the exemplary embodiments of **FIGS. 8A** and **8B** and **FIG. 10B**, the first microcolumn **112** is shown as having a first or upper extraction system or layered construction located in the upper full diameter bed region **130** comprised of: (i) upper flow distributor **116a** configured as a screen or mesh, (ii) upper compression layer **118a** such as a glass fiber filter material, (iii) optional sorbent or extraction layer **119**, and (iv) optional frit **117.** Furthermore, the upper or first microcolumn **112** also includes an additional extraction system or layered construction in the lower reduced diameter bed region **131**, here shown much like that of the embodiment of **FIG. 7B** wherein there is provided: (i) middle compression layer **118c**, (ii) air gap layer **114** (rather than an extraction layer **14** as in **FIGS. 1B** and **3**), and (iii) lower compression layer **118b.** It will be appreciated that the space between the upper extraction system or layered construction, here including an optional lowest layer as frit **117**, and the top of the lower extraction system or layered construction, here being the middle compression layer **118c**, defines yet another air gap layer analogous to the air gap layer **301** of **FIG. 7B****.** Thus, there are effectively two air gap layers in the upper or first microcolumn **112** alone in this alternative exemplary embodiment. Once again, those skilled in the art will appreciate that the number, arrangement, and actual or proportional size of any such air gaps are merely illustrative of aspects of the present invention and non-limiting. It is again noted in connection with **FIG. 11B** that, though not shown, an optional lower flow distributor/support analogous to such distributor **16c** shown in **FIG. 7B** may be molded as part of the container or otherwise provided as part of the lower reduced diameter bed region **131** of the upper or first microcolumn **112.** Again, additional layers may be added or substituted, it being noted, for example, the alternative position of the optional frit **117** and the inclusion of an upper sorbent **119** rather than a second flow distributor layer (compare to middle flow distributor **16b** of **FIGS. 1B****,** **3****,** **7A, 7B****,** **8A** and **8B**).

And in **FIG. 11C** there is again shown an enlarged partial cross-sectional view of a lower or second microcolumn **212** associated with the lower or second stage or array **270** of the apparatus **110** shown in **FIG. 11A**, as taken along section line **11C-11C of** **FIG. 11A****.** For simplicity, the second microcolumn **212** of **FIG. 11C** is shown as being identical to that of **FIG. 10C**, though it will be appreciated that other such configurations of the lower or second extraction system or layered construction located in the lower reduced diameter bed region **231**, with or without an extraction system or layers in the upper full diameter bed region **230**, may be employed without departing from the scope of the invention. In the illustrated embodiment, once again, the lower second layered construction is comprised of: (i) middle compression layer **218c**, (ii) extraction layer **214** of microparticulate extraction medium, and (iii) lower compression layer **218b**, with additional layers being added or substituted depending on the context. As also again shown in **FIG. 11C**, optionally, microcolumn passage **223** may be tapered from the upper full diameter bed region **230** to the reduced diameter bed region **231** to assist in transitioning the sample or elution liquid from one bed region to the other.

Regarding the alternative embodiment of the apparatus **110** as shown in **FIGS. 11A-****11C**, it will again be appreciated that there are provided two stages with two separate microcolumns **112**, **212** in series, the upper first microcolumn **112** being configured with a layered construction at least in its upper full diameter bed region **130** and the lower second microcolumn **212** being configured with a layered construction in its lower reduced diameter bed region **231.** Accordingly, there is once more achieved a multi-stage extraction system as generally shown and described herein with at least two distinct bed regions and related layered constructions, or effectively two stages, again doing so in two microcolumns **112**, **212** in series. As noted above in connection with the alternative upper or first microcolumn **112** of the present embodiment, by including spaced apart middle and lower compression layers **118c**, **118b** there are provided two air gaps in the first microcolumn **112** alone, and again, by physically separating the upper and lower extraction or filtration systems between the two microcolumns **112**, **212**, larger volumes or spaces therebetween are formed that further serve as air gaps, all such air gaps cooperating to prevent or mitigate against dripping or capillary flow and to allow for the transfer of the one or more columns between each other or to an appropriate receiving vessel or plate. Moreover, in the exemplary arrangement of the upper or first microcolumn **112** as shown in **FIG. 11B**, a further alternative embodiment might be to include an extraction layer or sorbent analogous to extraction layer **14** in **FIGS. 1B****,** **3** and **9A** rather than the air gap **114** shown in **FIG. 11B****.** It will be appreciated that the result in this alternative exemplary embodiment would be effectively three extraction systems or a three-stage solid-phase extraction system achieved using two of the novel microcolumns **112**, **212** in series according to aspects of the present invention, with two such stages occurring in the upper first microcolumn **112** and an effective third stage in the lower second microcolumn **212.** It will be further appreciated that a four-stage arrangement could be provided by also including an extraction system in the upper full diameter bed region **230** of the lower or second microcolumn **212**, or there may be only one stage in the upper first microcolumn **112** as shown in **FIGS. 10B** and **11B** and instead two stages in the lower second microcolumn **212.** Again, those skilled in the art will appreciate that any such arrangements or configurations are possible without departing from the scope of the invention, including having two or more stages and two or more microcolumns in series, with the stages distributed among the microcolumns in a wide variety of arrangements. In that regard, there is once more thus provided new functional utility and increased fields of use in the extraction apparatus **110** according to aspects of the present invention, whereby relatively more effective extraction as measured by absolute recovery or otherwise is possible even using the same or smaller elution volume, resulting in less dead or unused volume and less contaminates in the extracted analytes.

Although the present invention has been described in considerable detail with reference to certain preferred versions thereof, other versions are possible. For example, the apparatus **10** is not limited to use with biological fluids, but can be used, for example, for testing ground water, drinking water, and other liquids for contaminants.

### EXAMPLES

The following non-limiting examples are provided for illustrative purposes only in order to facilitate a more complete understanding of the disclosed subject matter. These examples should not be construed to limit any of the embodiments described in the present specification, including those pertaining to the apparatus used therein and the methods of using the present apparatus.

### Example 1

### Extraction of the Catecholamines from Plasma

Control Single diameter column: CEREX® WCX 1cc 10 mg columns.

Present Apparatus "narrow bore column": The narrow bore column was a 1 cc column having an effective area ratio of 4:1 (full diameter to reduced diameter). The layers of the column were: (i) an upper flow distributor screen, (ii) a cylindrical fabric compression layer, (iii) a lower flow distributor screen, (iv) a spherical frit as a compression layer, (v) the microparticulate extraction medium, and (vi) a spherical frit as a lower compression layer. Layers (i)-(iii) were located in the full diameter region, where layers (iv)-(vi) were located in the reduced diameter region. The extraction medium was the same as the extraction medium used in the CEREX® WCX 1cc 10 mg columns.

Normal human serum samples were obtained from Bioreclamation Corp. and spiked with catecholamines prior to solid phase extraction. The blanks and plasma samples were also spiked with internal standards (e.g., dopamine-D4, epinephrine-D6, and norepinephrine-D6).

CEREX® WCX (1cc /10mg) (control and narrow bore) columns were conditioned with 0.5ml of methanol, followed by 0.5ml of 10mM Phosphate Buffer pH 6.8.

0.5 mL 10 mM Phosphate buffer was mixed with 100 µL of the Sample. The Sample/buffer mix at a pH of 6.8 was loaded onto the column at a pressure of 2-3 psi. The column was washed with 1 mL deionized water at 6 psi and subsequently washed with 1ml Acetonitrile at 6 psi.

Two sets of columns were loaded for each type of column. One half of the columns (control and narrow bore) were eluted with 0.5 mL of Elution Buffer - 25:75 100 mM Potassium Carbonate:Acetonitrile. The other half of the columns were eluted with 0.1 mL of 25:75 100 mM Potassium Carbonate:Acetonitrile.

25 µL of the eluent was used or loaded for the LC-MS/MS reaction.

### Example 2

### LC-MS/MS Analysis of the Catecholamines

25 µL of the solution obtained from the extraction was automatically injected into a TARGA® C18 3µm particle size 50 x 2.1 mm analytical column. A binary HPLC gradient was applied to the analytical column to separate the epinephrine, norepinephrine and dopamine from other analytes contained in the sample. Mobile phase A was 5.0 mM ammonium formate with 0.1% formic acid pH 3.0 and mobile phase B was Acetonitrile with 0.1% formic acid. The HPLC gradient proceeded at a temperature of 35°C with a flow rate of 500 µL/min over five minutes as follows:

### Gradient:

| **Time (min)** | **B (%)** |
|---|---|
| 0.01 | 50 |
| 3.00 | 100 |
| 4.00 | 100 |
| 4.50 | 50 |
| 5.00 | 50 |

MS/MS was performed using an APPLIED BIOSYSTEMS MDS SCIEX 500®, although other suitable MS/MS apparatuses are known. The following software programs were used in the present examples: ANALYST 1.5.2®, although other suitable software systems are known. Liquid solvent/analyte exiting the analytical column flowed to the heated nebulizer interface of the MS/MS analyzer. The solvent/analyte mixture was converted to vapor in the heated tubing of the interface. Analytes in the nebulized solvent were ionized by heated ESI.

Ions passed to the first quadrupole (Q1), which selected ions with a mass to charge ratio of parent ions generated from one of the analytes. Ions entering quadrupole 2 (Q2) collided with argon gas to generate ion fragments, which were passed to quadrupole 3 (Q3) for further selection. After measurement of ions indicative of one of the analytes, Q1 was adjusted so that ions with a mass to charge ratio of parent ion from a second analyte were selected. These ions were collided with nitrogen gas in Q2, and the ion fragments passed to Q3 for further selection. After measurement of these ions, Q1 was adjusted so that ions with a mass to charge ratio of parent ion from a third analyte were selected. These ions were collided with argon gas in Q2, and the ion fragments passed to Q3 for further selection. Simultaneously, the same process using isotope dilution mass spectrometry was carried out with internal standards, dopamine-D4 and/or epinephrine-D6, and/or norepinephrine-D6. The following mass transitions were used for detection and quantitation of epinephrine, norepinephrine and dopamine (and their corresponding internal standards) during validation on positive polarity from the same sample injection.

Table 1 shows the data representing the percent recovery of catecholamines from a plasma sample spiked with 1 ng/ml catecholamine. Notably, the narrow bore columns performed significantly better for all catecholamines at all elution volumes. In particular, the small volume elution (i.e., the 0.1 ml elution) performed at least approximately three times as well as the small volume elution using the conventional column.

| **Table 1: Absolute Recovery Comparison at 1ng/ml Plasma** | | | | |
|---|---|---|---|---|
| Compound | Cerex 10mg 1cc WCX column 0.5ml elution | Cerex 10mg 1cc WCX column 0.1ml elution | Cerex 10mg 1cc WCX Narrow Bore 0.5ml elution | Cerex 10mg 1cc WCX Narrow Bore 0.1ml elution |
| Dopamine | 98.3 % cv= 8.3% | 27.6% cv=11.5% | 99.5% cv=4.6% | 99.6% cv= 4.4% |
| Dopamine-D4 | 96.8 % cv= 9.8% | 32.4% cv=10.4% | 99.5% cv=4.2% | 98.6% cv= 4.7% |
| Epinephrine | 92.3% cv= 12.6% | 35.6% cv=11.5% | 97.5% cv=3.8% | 97.2% cv= 5.0% |
| Epinephrine-D6 | 91.4 % cv= 12.8% | 38.5% cv=13.2% | 96.7% cv=4.1% | 98.1% cv= 4.5% |
| Norepinephrine | 87.3% cv= 14.3% | 15.6% cv=22.5% | 93.0% cv=5.8% | 92.1% cv= 6.2% |
| Norepinehrine-D6 | 85.6% cv=15.2% | 13.9% cv=23.0% | 91.5% cv=5.4% | 90.9% cv= 5.7% |

As shown in the chromatograms of **FIGS. 4A-F** and **FIGS. 5A-F**, extracting and detecting catecholamine in plasma from healthy donors by using the present apparatus (i.e., the narrow bore column) is plotted over time.

The chromatography demonstrates the sensitivity of the assay. The absolute recovery chart demonstrates the improved recovery and improved reproducibility using the narrow bore columns vs conventional columns, even at smaller elution volumes.

Another benefit of the present apparatus is that due to the smaller elution volumes from the reduced effective bed diameter the present apparatus can collect into smaller vessels and eliminate or reduce transfers.

### Example 3

### Extraction of Buprenorphine and Norbuprenorphine from Urine

Narrow Bore Extraction columns feature high-capacity, high-efficiency, low bed mass sorbents that permit the use of low elution volumes (50-100 µL). These elution volumes lend themselves to evaporation within the positive pressure SPE processor unit such as an ALDIII™ or an IP8™ (SPEware Corp., Baldwin Park, CA), eliminating the need for a separate solvent evaporator. This allows the use of selective elution solvents, resulting in cleaner extracts than would be possible using high solvent strength (low specificity) elution solvents.

Buprenorphine and norbuprenorphine were chosen as model compounds for several reasons. They are frequently monitored as part of "pain panels" in compliance testing laboratories. Their relevant concentrations are relatively low and thus require low LLODs. Additionally, they are excreted in urine primarily as glucuronide conjugates, affording the opportunity to evaluate both solid phase extraction efficiency with narrow bore SPE columns.

### Experimental Reagents

a) water, b) negative control urine (diluent for the standard curve), and c) a "master mix" containing 100 mM sodium acetate buffer pH 4.8, the internal standard solution (B-d4 and N-d3), and β-glucuronidase solution (2500 units/sample, catalog #BG100, red abalone, Kura Biotec, Inglewood, CA).

### Process

A calibration curve was prepared from a single high calibrator via serial dilution. "Master mix" was placed in all wells of a 96-well incubation plate on the plate heater (preheated to 68 °C). Calibrators and controls (100 µL specimen volume) were then transferred to the incubator plate. After 15 minutes, the contents of the incubation plate were transferred to the 96-well SPE plate for extraction.

### Solid phase extraction:

Apply samples to narrow bore extraction columns with PSCX sorbent (2.5 mg)
Wash w/ 250 µL deionized water
Wash w/ 150 µL 100 mM acetic acid
Wash w/ 300 µL methanol
Dry sorbent for 1 minute
Transfer the SPE plate to collection
Elute w/ 50 µL elution solvent (ethyl acetate: methanol:conc. NH4OH=93:5:2)
Dry the solvent
Dissolve residues in the reconstitution solvent (100mL, aqueous formic acid 0.1% : methanol = 80:20)

### Analytical Conditions

### LC Conditions:

Column: Haisil C18 HL, 50x2.1 mm, 5µM (Higgins Analytical, Inc., Mountain View, CA)
Flow: 400µL/min
Injection volume: 10µL
A=0.1% Aqueous formic acid; B=Methanol;
Gradient: 20-40% B in 0.5 min., 40-60% B in 2 min.
MS Conditions: Sciex 5000, Source=ESI; Positive ion MRM
Buprenorphine 468.25->55.10 (quant), 83.2(qual)
Buprenorphine-d4 472.42->59.0 (quant), 83.0 (qual)
Norbuprenorphine 414.200->55.10 (quant), 83.0 (qual)
Norbuprenorphine-d3 417.01->54.8 (quant), 83.1 (qual)

### Results and Conclusions

Calibration curves for B and N are shown in **FIGS. 6A-B**. Regression is quadratic, 1/x weighted. Based on s/n ratios of the quantitation ions and assessment of curve accuracy (requiring ± 20% from nominal value at the low calibrator), the LLOQs were determined to be 0.313 ng/mL and 0.625 for B and N, respectively.

Results from the analysis of non-validated control samples (n=10 each) are as follows:

| **Table 2: Burprenorphine** | | | | | | | |
|---|---|---|---|---|---|---|---|
| Buprenorphine | | | | Hydrolysis | | Hydrolysis | |
| Low Control #1 | | Low Control #2 | | Control | Low Control #1 | Low Control #2 | Control |
| 0.75 ng/mL | | 1.2 ng/mL | | 5.0 ng/mL | 100 ng/mL | 150 ng/mL | 100 ng/mL |
| Average | 0.91 | | 1.4 | 5.3 | 107 | 167 | 110 |
| Bias | 22% | | 16% | 6% | 7% | 11% | 10% |
| CV (RSD) | 3.1% | | 3.1% | 3.6% | 3.2% | 4.0% | 3.6% |

| **Table 3: Norbuprenorphine** | | | | | | | |
|---|---|---|---|---|---|---|---|
| Norbuprenorphine | | | | Hydrolysis | | Hydrolysis | |
| Low Control #1 | | Low Control #2 | | Control | Low Control #1 | Low Control #2 | Control |
| 0.75 ng/mL | | 1.2 ng/mL | | 5.0 ng/mL | 100 ng/mL | 150 ng/mL | 100 ng/mL |
| Average | 0.97 | | 1.4 | 4.7 | 106 | 165 | 103 |
| Bias | 29% | | 16% | -6% | 6% | 10% | 3% |
| CV (RSD) | 3.5% | | 4.0% | 4.8% | 2.7% | 4.0% | 5.6% |

RSDs were less than 5%. Absolute recoveries from urine using the Narrow Bore Extraction column with a 2.5 mg bed mass were 91% and 97% for B and N, respectively. The experiment was repeated with a urine sample supplemented with morphine at 20,000 ng per mL. No difference in absolute recovery was noted.

### Example 4

### Extraction of Benzoylecgonine, Buprenorphine, EDDP, and Meprobamate from Urine

The following experiment compares extraction recovery of four (4) common analytes in urine with regular CEREX HPSCX NBE columns and a set of dual stage SPE columns with filtration only on the first stage and HPSCX sorbent in the narrow bore second stage without top filtration layers.

A blank urine sample was spiked with 100ng of benzoylecgonine, buprenorphine, EDDP, and meprobamate with their deuterated internal standards.

### Sample preparation

### Regular NBE columns:

Urine sample (50uL) was mixed with 100mM sodium acetate (aq., pH 4.8, 250uL) and loaded into the NBE SPE columns. Washing the columns with 100mM HCI (300 uL), then DI water (500 uL), dry the columns with a stream of nitrogen for 10 minutes. Elute the sample with the elution buffer (DCM:IPA:NH4OH=80:18:2, 50uL). The eluted samples were dried over nitrogen. The residue was reconstituted with 200 µL of a mixture of 0.1% aqueous formic acid:methanol=95:5 and spiked with IS. The samples were analyzed via LCMS.

### Solid phase extraction with dual stage NBE columns:

Urine samples (50uL) was mixed with 100mM sodium acetate (aq., pH 4.8, 250uL) and loaded into the dual stage NBE columns. The samples were applied to the second stage columns through the first columns. The first stage columns were removed. The samples were loaded onto the sorbent in the second stage columns. The columns with sorbent were washed with 100mM HCI (300uL), then DI water (500uL). Columns were dried through a stream of nitrogen for 10 minutes. The residue was reconstituted with 100 µL of a mixture of 0.1% aqueous formic acid:methanol=90:10 and skiped with IS. The samples were analyzed via LCMS.

### Process

### LCMS method:

Analysis of the extracts was performed on a SCIEX 5500 mass spectrometer interfaced to a Shimadzu Nexera XR UHPLC. The acquisition program was built using scheduled MRM (two product ions for each analyte, on product ion for each internal standard).

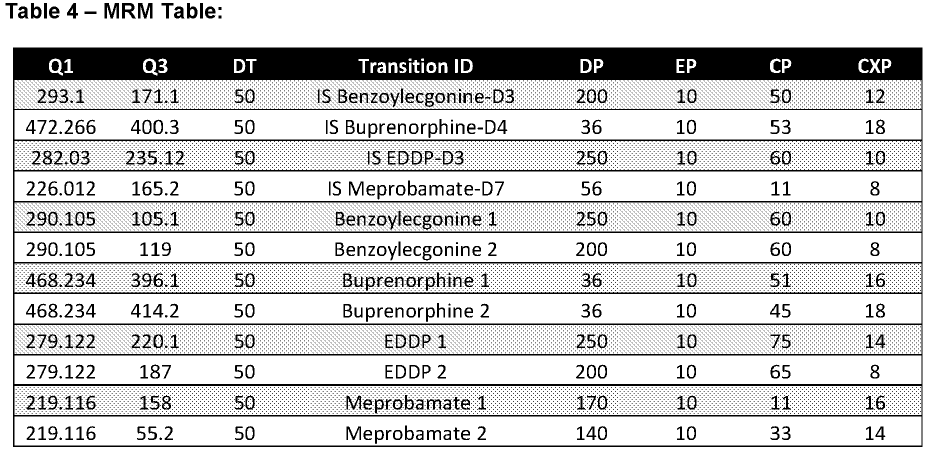

### Analytical Conditions

### MS Conditions:

SCIEX QTRAP® 5500, Source=ESI
Positive ion, scheduled MRM
Source temperature 600 °C
Desolvation GS1, GS2: 50
HPLC gradient profile:

| Time | % B |
|---|---|
| 0.00 | 5% |
| 4.00 | 80% |
| 4.20 | 100% |
| 4.70 | 100% |
| 4.71 | 5% |
| 5.71 | 5% |

Mobile Phase A 0.1% Formic acid (aqueous)
Mobile Phase B MeOH + 0.1% Formic Acid
Flow Rate : 0.7mL/min
Injection Volume 5ul

### LC Conditions:

### Shimadzu Nexera XR UHPLC

Column: RaptorTM Biphenyl, 50mm x 2.1mm, 2.7um, with Raptor Biphenyl 5mm guard column, Restek Corp., Bellefonte, PA
Flow: 700 µL/min
Column temperature: 40 °C (SPEware column oven)
Injection volume: 5 µL
A=0.1% aqueous formic acid; B=0.1% formic acid in Methanol

### Results and Conclusions

The following results showed that except for buprenorphine, SPE with the dual stage NBE device provided significantly better recovery than the regular NBE columns with 50uL of elution solvent.

**Table 5 - Experimental results for four analytes**

| **Sample Name** | **Component Name** | **Area** | **IS Area** | **% Recovery** |
|---|---|---|---|---|
| Std R1 | Benzoylecgonine 1 | 786115 | 552071 | 100 |
| 50ul elution Dual stage HPSCX | Benzoylecgonine 1 | 616656 | 538764 | 80 |
| 50ul elution NBE HPSCX | Benzoylecgonine 1 | 599230 | 571348 | 74 |
| Std R1 | Benzoylecgonine 2 | 201261 | 552071 | 100 |
| 50ul elution Dual stage HPSCX | Benzoylecgonine 2 | 165762 | 538764 | 84 |
| 50ul elution NBE HPSCX | Benzoylecgonine 2 | 163685 | 571348 | 79 |
| Std R1 | Buprenorphine 1 | 351802 | 304632 | 100 |
| 50ul elution Dual stage HPSCX | Buprenorphine 1 | 302683 | 234453 | 112 |
| 50ul elution NBE HPSCX | Buprenorphine 1 | 343104 | 254600 | 117 |
| Std R1 | Buprenorphine 2 | 320771 | 304632 | 100 |
| 50ul elution Dual stage HPSCX | Buprenorphine 2 | 288572 | 234453 | 117 |
| 50ul elution NBE HPSCX | Buprenorphine 2 | 332709 | 254600 | 124 |
| Std R1 | EDDP 1 | 1196197 | 319077 | 100 |
| 50ul elution Dual stage HPSCX | EDDP 1 | 937635 | 206197 | 121 |
| 50ul elution NBE HPSCX | EDDP 1 | 612008 | 236699 | 69 |
| Std R1 | EDDP 2 | 922362 | 319077 | 100 |
| 50ul elution Dual stage HPSCX | EDDP 2 | 742065 | 206197 | 125 |
| 50ul elution NBE HPSCX | EDDP 2 | 497855 | 236699 | 73 |
| Std R1 | Meprobamate 1 | 609769 | 477638 | 100 |
| 50ul elution Dual stage HPSCX | Meprobamate 1 | 428435 | 445650 | 75 |
| 50ul elution NBE HPSCX | Meprobamate 1 | 395076 | 510502 | 61 |
| Std R1 | Meprobamate 2 | 1354992 | 477638 | 100 |
| 50ul elution Dual stage HPSCX | Meprobamate 2 | 1046582 | 445650 | 83 |
| 50ul elution NBE HPSCX | Meprobamate 2 | 1008428 | 510502 | 70 |

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings. Lastly, the terminology used herein is for the purpose of describing particular embodiments only, and is not intended to limit the scope of the present invention, which is defined solely by the claims. Accordingly, the present invention is not limited to that precisely as shown and described.

Certain embodiments of the present invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the present invention to be practiced otherwise than specifically described herein. Accordingly, this invention includes all modifications and equivalents of the subject matter recited in the claims appended hereto as permitted by applicable law. Moreover, any combination of the above-described embodiments in all possible variations thereof is encompassed by the invention unless otherwise indicated herein or otherwise clearly contradicted by context.

Groupings of alternative embodiments, elements, or steps of the present invention are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the specification and attached claims are approximations that may vary. For instance, as mass spectrometry instruments can vary slightly in determining the mass of a given analyte, the term "about" in the context of the mass of an ion or the mass/charge ratio of an ion refers to +/-0.50 atomic mass unit.

At the very least, and not as an attempt to limit the application of the doctrine of equivalents to the scope of the claims, each numerical indication should at least be construed in light of the number of reported significant digits and by applying ordinary rounding techniques.

Use of the terms "may" or "can" in reference to an embodiment or aspect of an embodiment also carries with it the alternative meaning of "may not" or "cannot." As such, if the present specification discloses that an embodiment or an aspect of an embodiment may be or can be included as part of the inventive subject matter, then the negative limitation or exclusionary proviso is also explicitly meant, meaning that an embodiment or an aspect of an embodiment may not be or cannot be included as part of the inventive subject matter. In a similar manner, use of the term "optionally" in reference to an embodiment or aspect of an embodiment means that such embodiment or aspect of the embodiment may be included as part of the inventive subject matter or may not be included as part of the inventive subject matter. Whether such a negative limitation or exclusionary proviso applies will be based on whether the negative limitation or exclusionary proviso is recited in the claimed subject matter.

Notwithstanding that the numerical ranges and values setting forth the broad scope of the invention are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present specification as if it were individually recited herein.

The terms "a," "an," "the" and similar referents used in the context of describing the present invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the present invention so claimed are inherently or expressly described and enabled herein.

## Claims

1. An apparatus (10) for extracting an analyte from a liquid sample, the apparatus (10) comprising:
a) an upper first microcolumn (112) and a lower second microcolumn (212) in series, allowing flowing of the sample from the first microcolumn (112) into the second microcolumn (212), each microcolumn (112;212) having an entrance (20), an exit (22), and a passage (23) therebetween for passage of a liquid sample containing an analyte therethrough, whereby the first microcolumn (112) defines a first passage and the second microcolumn (212) defines a second passage, each microcolumn (112;212) further having a full diameter bed region (130;230) nearest the entrance (20), a reduced diameter bed region (131;231) between the full diameter bed region (130;230) and the exit (22), and a tip region (33) nearest the exit (22), a first shelf (52) defined by a step in diameter between the full diameter bed region (130;230) and the reduced diameter bed region (131;231), and a second shelf (54) defined by a step in diameter between the reduced diameter bed region (131;231) and the tip region (33);
b) a first layered construction having a top and a bottom extending across the first passage of the first microcolumn (112) and being positioned atop its first shelf (52), comprising from top to bottom: (i) an upper mesh flow distributor (116a) and (ii) an upper compression layer (118a), the first layered construction located in the full diameter bed region (130) of the first microcolumn (112); and
c) a second layered construction having a top and a bottom extending across the second passage of the second microcolumn (212) and being positioned atop its second shelf (54), comprising from top to bottom: (iii) an extraction layer (214) of microparticulate extraction medium and (iv) a lower compression layer (218b), the second layered construction located in the reduced diameter bed region (231) of the second microcolumn (212).

2. The apparatus (10) of claim 1 wherein the first layered construction further comprises an extraction layer (119) beneath the upper compression layer (118a).

3. The apparatus (10) of claim 1 comprising a third layered construction having a top and a bottom extending across the first passage of the first microcolumn (112), comprising from top to bottom: (i) a middle compression layer (118c) and (ii) a lower compression layer (118b), the third layered construction located in the reduced diameter bed region (131) of the first microcolumn (112).

4. The apparatus of claim 3 wherein the third layered construction further comprises an air gap (114) between the middle compression layer (118c) and the lower compression layer (118b).

5. The apparatus of claim 3 wherein the third layered construction further comprises an extraction layer (14) between the middle compression layer (118c) and the lower compression layer (118b).

6. A method for the extraction of an analyte from a sample comprising:
a) contacting an apparatus (10) of claim 1 with a sample in a liquid buffer; and
b) eluting the sample from the apparatus (10) with an elution buffer.

7. The method of claim 6 wherein the step of contacting the apparatus (10) comprises filtering the sample through the first microcolumn (112) under pressure and flowing the sample into the second microcolumn (212) under reduced pressure, ventilation is provided by unsealingly nesting the first microcolumn (112) within the second microcolumn (212).

8. The method of claim 7 wherein the step of contacting the apparatus (10) further comprises removing the first microcolumn (112) and filtering the sample through the second microcolumn (212) and the step of eluting the sample from the apparatus (10) comprises flowing the elution buffer through the second microcolumn (212).

## Patentansprüche

1. Vorrichtung (10) zum Extrahieren eines Analyten aus einer flüssigen Probe, wobei die Vorrichtung (10) umfasst:
a) eine obere erste Mikrosäule (112) und eine untere zweite Mikrosäule (212) in Serie, welche das Fliessen der Probe von der ersten Mikrosäule (112) in die zweite Mikrosäule (212) ermöglichen, wobei jede Mikrosäule (112;212) einen Eingang (20), einen Ausgang (22) und dazwischen einen Durchgang (23) zum Passieren einer flüssigen Probe hat, die einen Analyten enthält, wobei die erste Mikrosäule (112) einen ersten Durchgang und die zweite Mikrosäule (212) einen zweiten Durchgang begrenzt, wobei jede Mikrosäule (112;212) ferner einen Bettbereich mit vollem Durchmesser (130;230) aufweist, der dem Eingang (20) am nächsten liegt, einen Bettbereich mit reduziertem Durchmesser (131;231) aufweist, der zwischen dem Bettbereich mit vollem Durchmesser (130;230) und dem Ausgang (22) liegt und ein Spitzenbereich (33) aufweist, der dem Ausgang (22) am nächsten liegt, wobei ein erster Absatz (52) durch eine Durchmesserstufe zwischen dem Bettbereich mit vollem Durchmesser (130;230) und dem Bettbereich mit reduziertem Durchmesser (131;231) definiert ist und wobei ein zweiter Absatz (54) durch eine Durchmesserstufe zwischen dem Bettbereich mit reduziertem Durchmesser (131; 231) und dem Spitzenbereich (33) definiert ist;
b) einen ersten Schichtaufbau mit einer Oberseite und einer Unterseite, welcher sich durch den ersten Durchgang der ersten Mikrosäule (112) erstreckt und welcher auf dessen erstem Absatz (52) positioniert ist und von oben nach unten umfasst: (i) einen oberen Maschenströmungsverteiler (116a) und (ii) eine obere Kompressionsschicht (118a), wobei sich der erste Schichtaufbau im Bettbereich mit vollem Durchmesser (130) der ersten Mikrosäule (112) befindet; und
c) einen zweiten Schichtaufbau mit einer Oberseite und einer Unterseite, welcher sich durch den zweiten Durchgang der zweiten Mikrosäule (212) erstreckt und welcher auf dessen zweitem Absatz (54) positioniert ist und von oben nach unten umfasst: (iii) eine Extraktionsschicht (214) aus mikropartikulärem Extraktionsmedium und (iv) eine untere Kompressionsschicht (218b), wobei sich der zweite Schichtaufbau im Bettbereich mit reduziertem Durchmesser (231) der zweiten Mikrosäule (212) befindet.

2. Vorrichtung (10) nach Anspruch 1, wobei der erste Schichtaufbau ferner eine Extraktionsschicht (119) unterhalb der oberen Kompressionsschicht (118a) umfasst.

3. Vorrichtung (10) nach Anspruch 1, umfassend einen dritten Schichtaufbau mit einer Oberseite und einer Unterseite, welcher sich durch den ersten Durchgang der ersten Mikrosäule (112) erstreckt und von oben nach unten umfassen: (i) eine mittlere Kompressionsschicht (118c) und (ii) eine untere Kompressionsschicht (118b), wobei sich der dritte Schichtaufbau im Bettbereich mit reduziertem Durchmesser (131) der ersten Mikrosäule (112) befindet.

4. Vorrichtung nach Anspruch 3, wobei der dritte Schichtaufbau ferner einen Luftspalt (114) zwischen der mittleren Kompressionsschicht (118c) und der unteren Kompressionsschicht (118b) umfasst.

5. Vorrichtung nach Anspruch 3, wobei der dritte Schichtaufbau ferner eine Extraktionsschicht (14) zwischen der mittleren Kompressionsschicht (118c) und der unteren Kompressionsschicht (118b) umfasst.

6. Verfahren zur Extraktion eines Analyten aus einer Probe, umfassend:
a) Inkontaktbringen einer Vorrichtung (10) nach Anspruch 1 mit einer Probe in einem flüssigen Puffer; und
b) Eluieren der Probe aus der Vorrichtung (10) mit einem Elutionspuffer.

7. Verfahren nach Anspruch 6, wobei der Schritt des Inkontaktbringens der Vorrichtung (10), das Filtern der Probe durch die erste Mikrosäule (112) unter Druck und das Fließen der Probe in die zweite Mikrosäule (212) unter vermindertem Druck umfasst, wobei eine Belüftung bereitgestellt wird, indem die erste Mikrosäule (112) nicht dichtend innerhalb der zweiten Mikrosäule (212) aufgenommen ist.

8. Verfahren nach Anspruch 7, wobei der Schritt des Inkontaktbringens der Vorrichtung (10), ferner das Entfernen der ersten Mikrosäule (112) und das Filtern der Probe durch die zweite Mikrosäule (212) umfasst, und wobei der Schritt des Eluierens der Probe von der Vorrichtung (10) das Fliesen des Elutionspuffers durch die zweite Mikrosäule (212) umfasst.

## Revendications

1. Appareil (10) pour extraire un analyte d'un échantillon liquide, l'appareil (10) comprenant:
a) une première microcolonne supérieure (112) et une deuxième microcolonne inférieure (212) en série, permettant l'écoulement de l'échantillon de la première microcolonne (112) dans la deuxième microcolonne (212), chaque microcolonne (112;212) ayant une entrée (20), une sortie (22) et un passage (23) entre eux pour le passage d'un échantillon liquide contenant un analyte à travers celui-ci, où la première microcolonne (112) définit un premier passage et la deuxième microcolonne (212) définit un deuxième passage , chaque microcolonne (112;212) ayant en outre une région de lit de diamètre complet (130;230) la plus proche de l'entrée (20), une région de lit de diamètre réduit (131;231) entre la région de lit de diamètre complet (130; 230) et le sortie (22), et une région de pointe (33) la plus proche de la sortie (22), une première étagère (52) définie par une étape de diamètre entre la région de lit de diamètre complet (130; 230) et la région de lit de diamètre réduit (131;231), et une deuxième étagère (54) définie par une étape de diamètre entre la région de lit de diamètre réduit (131;231) et la région de pointe (33) ;
b) une première construction en couches ayant un haut et un bas s'étendant à travers le premier passage de la première microcolonne (112) et étant positionnée au-dessus de sa première étagère (52), comprenant de haut en bas: (i) un distributeur d'écoulement à mailles supérieures (116a) et (ii) une couche de compression supérieure (118a), la première construction en couches située dans la région de lit de diamètre complet (130) de la première microcolonne (112); et
c) une deuxième construction en couches ayant un haut et un bas s'étendant à travers le deuxième passage de la deuxième microcolonne (212) et étant positionnée au-dessus de sa deuxième étagère (54), comprenant de haut en bas: (iii) une couche d'extraction (214) de milieu d'extraction microparticulaire et (iv) une couche de compression inférieure (218b), la deuxième construction en couches située dans la région de lit de diamètre réduit (231) de la deuxième microcolonne (212).

2. Appareil (10) selon la revendication 1, dans lequel la première construction en couches comprend en outre une couche d'extraction (119) sous la couche de compression supérieure (118a).

3. Appareil (10) selon la revendication 1, comprenant une troisième construction en couches ayant un haut et un bas s'étendant à travers le premier passage de la première microcolonne (112), comprenant de haut en bas: (i) une couche de compression médiane (118c) et (ii) une couche de compression inférieure (118b), la troisième construction en couches située dans la région de lit de diamètre réduit (131) de la première microcolonne (112).

4. Appareil selon la revendication 3, dans lequel la troisième construction en couches comprend en outre un espace d'air (114) entre la couche de compression médiane (118c) et la couche de compression inférieure (118b).

5. Appareil selon la revendication 3, dans lequel la troisième construction en couches comprend en outre une couche d'extraction (14) entre la couche de compression médiane (118c) et la couche de compression inférieure (118b).

6. Procédé d'extraction d'un analyte à partir d'un échantillon comprenant:
a) contacter un appareil (10) selon la revendication 1 avec un échantillon dans un tampon liquide; et
b) éluder l'échantillon de l'appareil (10) avec un tampon d'élution.

7. Procédé selon la revendication 6, dans lequel l'étape de contacter l'appareil (10) comprend filtrer l'échantillon à travers la première microcolonne (112) sous pression et écouler l'échantillon dans la deuxième microcolonne (212) sous pression réduite, une ventilation est fournie en emboîtant de manière non étanche de la première microcolonne (112) à l'intérieur de la deuxième microcolonne (212).

8. Procédé selon la revendication 7, dans lequel l'étape de contacter l'appareil (10) comprend en outre enlever la première microcolonne (112) et filtrer l'échantillon à travers la deuxième microcolonne (212) et l'étape d'éluder l'échantillon de l'appareil (10) comprend écouler le tampon d'élution à travers la deuxième microcolonne (212).
